(19) Europäisches Patentamt
European Patent Office
Office européen des brevets

(11) **EP 4 670 725 A1**

(12) **EUROPEAN PATENT APPLICATION**
published in accordance with Art. 153(4) EPC

(43) Date of publication:
**31.12.2025 Bulletin 2026/01**

(21) Application number: **24784904.5**

(22) Date of filing: **02.04.2024**

(51) International Patent Classification (IPC):
**A61K 35/744** (2015.01)   **A61P 27/00** (2006.01)
**A61P 31/14** (2006.01)

(52) Cooperative Patent Classification (CPC):
**A61K 35/744; A61P 27/00; A61P 31/14**

(86) International application number:
**PCT/JP2024/013573**

(87) International publication number:
**WO 2024/210119 (10.10.2024 Gazette 2024/41)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **03.04.2023 US 202363493874 P**

(71) Applicants:
• **NAGASAKI UNIVERSITY**
  **Nagasaki 852-8521 (JP)**
• **KIRIN HOLDINGS KABUSHIKI KAISHA**
  **Nakano-ku, Tokyo 164-0001 (JP)**

(72) Inventors:
• **YAMAMOTO Kazuko**
  **Nagasaki-shi, Nagasaki 852-8521 (JP)**
• **MUKAE Hiroshi**
  **Nagasaki-shi, Nagasaki 852-8521 (JP)**
• **JONAI Kenta**
  **Tokyo 164-0001 (JP)**
• **TSUJI Ryohei**
  **Tokyo 164-0001 (JP)**

(74) Representative: **Hoffmann Eitle**
**Patent- und Rechtsanwälte PartmbB**
**Arabellastraße 30**
**81925 München (DE)**

(54) **COMPOSITION FOR PREVENTING OR TREATING CHEMOSENSORY DISORDER**

(57)   The purpose of the present invention is to provide a composition for preventing or treating a chemosensory disorder. The present invention provides a composition for preventing or treating a chemosensory disorder, comprising a lactic acid bacterium. The chemosensory disorder can be a chemosensory disorder that occurs after SARS-CoV-2 viral infection. The present invention is advantageous in that lactic acid bacteria can be used as a functional ingredient that imparts a preventive or therapeutic effect against a chemosensory disorder, and a pharmaceutical product or a food product that is safe for mammals including humans can be provided.

**Fig.3**

EP 4 670 725 A1

## Description

### Reference to Related Application

The present application claims the benefit of priority from prior U.S. Provisional Application No. 63/493,874 (filed on April 3, 2023), the entire disclosure of which is incorporated herein by reference.

### Technical Field

The present invention relates to a composition for preventing or treating a chemosensory disorder.

### Background Art

COVID-19 (coronavirus disease-2019) remains a global public health threat, and the development of therapeutic drugs is an urgent task, but currently there are only a few effective therapeutic drugs. Actemra or baricitinib is used for severe COVID-19 patients, but there are currently limitations to the symptoms of eligible patients. In Japan, Zocova, an oral antiviral drug, was given emergency approval as a therapeutic drug (oral drug) for COVID-19 on November 22, 2022, but there are prescription restrictions due to concerns about teratogenicity, many interactions of the drug, or the high price of the drug. It has also been reported that COVID-19 patients experience chemosensory disorders such as taste disorders and olfactory disorders, and the problem is that the chemosensory disorders can persist for long periods of time even after symptoms such as fever have subsided. Preventive and therapeutic drugs for the chemosensory disorders have not yet been fully developed, and thus drugs that anyone can take or ingest are desired.

### Summary of Invention

An objective of the present invention is to provide a composition for preventing or treating a chemosensory disorder.

The present inventors have now discovered that the improvement of chemosensory disorders is accelerated in SARS-CoV-2-positive patients who have taken lactic acid bacteria. The present inventors have also discovered that the levels of pDCs in blood were maintained in a lactic acid bacteria intake group, and that the rates of reduction in SARS-CoV-2 viral load were accelerated in the lactic acid bacteria intake group. The present invention is based on these findings.

According to the present invention, the following inventions are provided.

[1] A composition for preventing or treating a chemosensory disorder, comprising a lactic acid bacterium.

[2] A method for preventing or treating a chemosensory disorder, comprising: administering a prophylactically or therapeutically effective amount of a lactic acid bacterium to a subject in need thereof.

[3] The composition according to [1] above or the method according to [2] above, wherein the chemosensory disorder is a chemosensory disorder occurring after SARS-CoV-2 viral infection.

[4] The composition according to [1] or [3] above or the method according to [2] or [3] above, wherein the chemosensory disorder is a taste disorder and/or an olfactory disorder.

[5] The composition according to any one of [1], [3] and [4] above or the method according to any one of [2], [3] and [4] above, wherein the chemosensory disorder is a chemosensory disorder occurring within 7 days after viral infection.

[6] The composition according to any one of [1] and [3] to [5] above, or the method according to any one of [2] to [5] above, wherein the lactic acid bacterium is a lactic acid bacterium activating pDCs and/or inducing the production of at least one type of IFN, and is preferably a *Lactococcus lactis* subsp. *lactis* JCM5805 strain.

[7] The composition according to any one of [1] and [3] to [6] above, wherein the composition is administered orally.

[8] The method according to any one of [2] to [6] above, wherein the lactic acid bacterium is administered orally.

[9] The composition according to [7] above or the method according to [8] above, wherein the dosage of the lactic acid bacterium is 20 mg or more and 2,000 mg or less per day.

[10] The composition according to [7] above or the method according to [8] above, wherein the dosage of the lactic acid bacterium is $1 \times 10^9$ or more cells and $4 \times 10^{11}$ or less cells per day.

[11] The composition according to any one of [1], [3] to [7], [9] and [10] above, which is a pharmaceutical composition or a food composition.

[12] Use of a lactic acid bacterium for the manufacture of the composition for preventing or treating a chemosensory disorder according to any one of [1], [3] to [7], [9] and [10] above.

[13] Use of a lactic acid bacterium in the method for preventing or treating according to any one of [2] to [6] and [8] to [10] above.

[14] Use of a lactic acid bacterium for preventing or treating a chemosensory disorder.

[15] A lactic acid bacterium for use in prevention or treatment of a chemosensory disorder.

The present invention is advantageous in that lactic acid bacteria can be used as a functional ingredient that imparts a preventive or therapeutic effect against chemosensory disorders, and pharmaceutical products or food products that are safe for mammals including humans can be provided. In particular, since there are only a limited number of drugs that are actively recommended for use in mildly ill COVID-19 patients who are not at risk of developing severe symptoms, the present invention is advantageous in that it can provide a preventive or therapeutic composition which can be taken safely, easily, and conveniently by such patients with mild symptoms.

**Brief Description of Drawings**

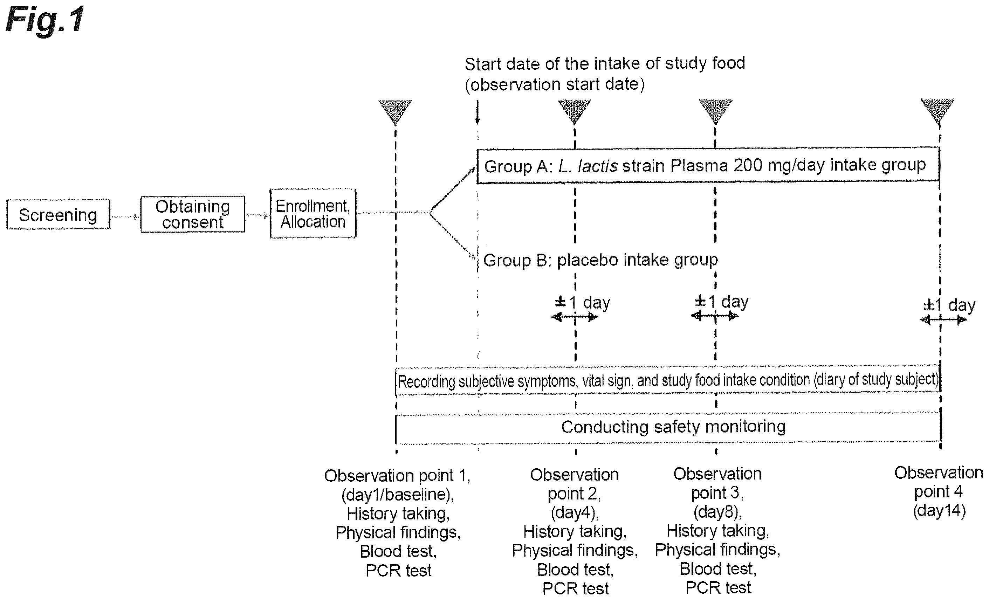

[FIG. 1] Fig. 1 is a chronological summary of the tests carried out in the examples.

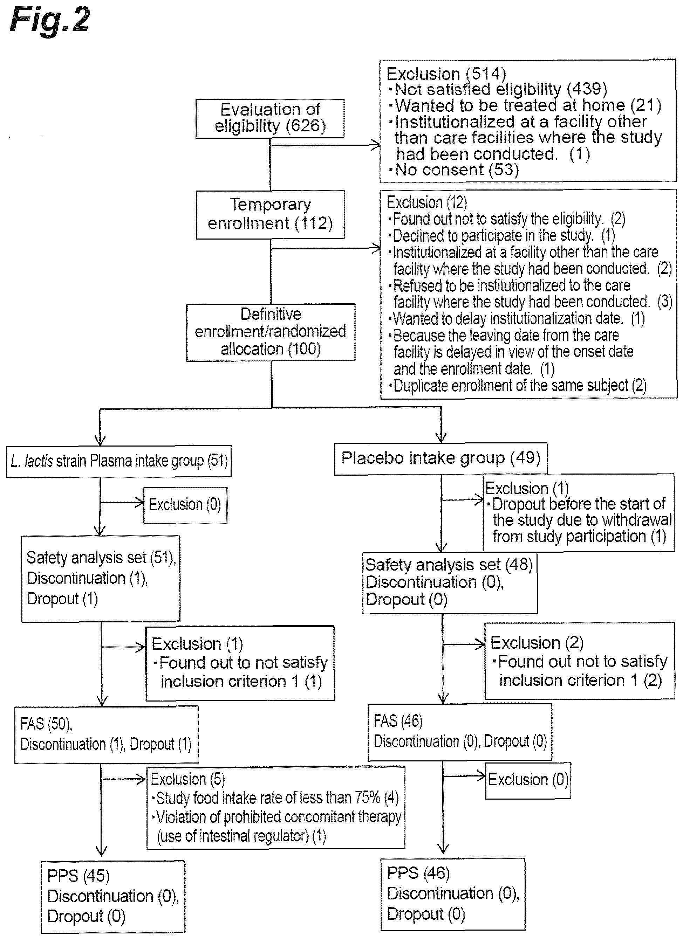

[FIG. 2] Fig. 2 is a diagram summarizing a flow chart of the tests carried out in the examples. Numbers in parentheses indicate the number of relevant cases.

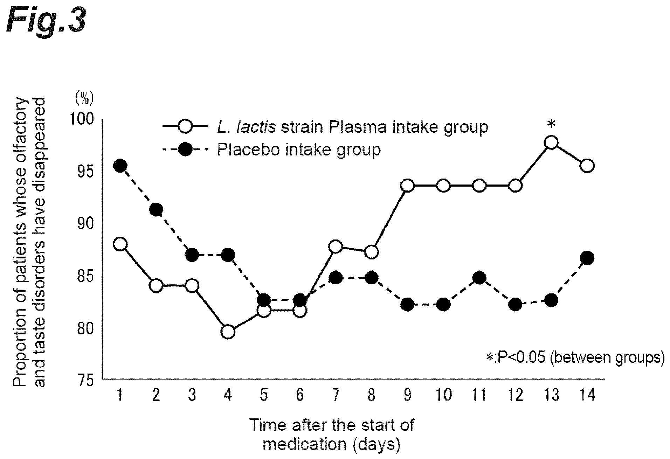

[FIG. 3] Fig. 3 is a diagram showing the proportion of patients over time, whose olfactory and taste disorders have disappeared, in a *L. lactis* strain Plasma intake group and the same of a placebo intake group, respectively, in the tests carried out in the examples. An increase in the proportion of patients whose olfactory and taste disorders have disappeared indicates improvement of symptoms.

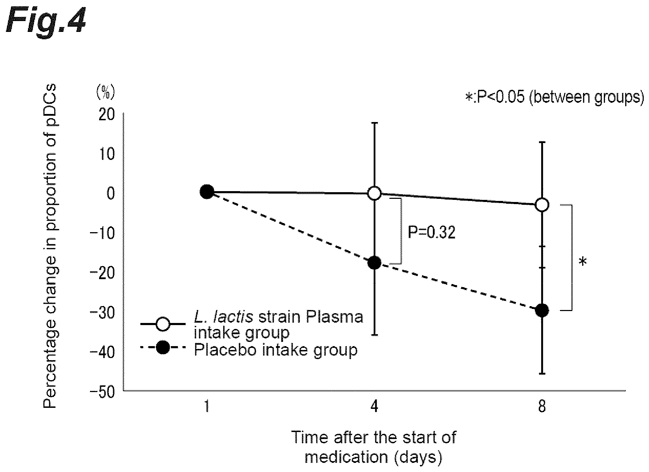

[FIG. 4] Fig. 4 is a diagram showing the percentage change over time in the proportion of pDCs in blood ((amount of change/measurement on day 1) $\times$ 100(%)) of a *L. lactis* strain Plasma intake group and the same of a placebo intake group, respectively, in the tests carried out in the examples. An increase in the percentage change in the proportion of pDCs in blood in the negative direction indicates a decrease in the amount of pDCs present.

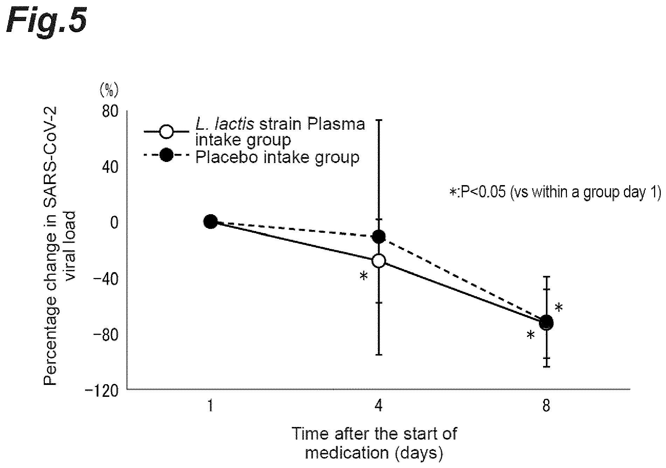

[FIG. 5] Fig. 5 is a diagram showing the percentage change over time of SARS-CoV-2 viral load obtained by the PCR method using nasopharyngeal swabs ((amount of change/measurement on day 1) $\times$ 100 (%)) of a *L. lactis* strain Plasma intake group and the same of a placebo group, respectively, in the tests carried out in the examples. An absolute value increase in the percentage change of SARS-CoV-2 viral load in the negative direction indicates a decrease in SARS-CoV-2 viral load.

**Description of Embodiments**

The active ingredient of the present invention, lactic acid bacteria, can activate pDC(s) and/or induce the production of at least one or more types of interferon (IFN).
The active ingredient of the present invention, lactic acid bacteria, can induce the production of at least one or more types of IFN: Type I IFN (type I interferon), Type II IFN (type II interferon) or Type III IFN (type III interferon). Type I IFN refers to a cytokine that is thought to be effective against viral infection, and examples thereof include IFN-$\alpha$ (including subtypes such as 1, 2, 4, 5, 6, 7, 8, 10, 13, 14, 16, 17, or 21) or IFN-$\beta$. Type II IFN includes IFN-$\gamma$ and Type III IFN includes IFN-$\lambda$. The active ingredient of the present invention, lactic acid bacteria, preferably has at least Type I IFN production-inducing activity. When pDCs are activated by the active ingredient of the present invention, lactic acid bacteria, cellular protrusions, which are characteristics of activated dendritic cells, appear and the production of Type I IFN and/or Type III IFN is induced, as well as the production of Type II IFN such as IFN-$\gamma$ from NK cells and Th1 cells can be induced.
IFN, the production of which can be induced by the active ingredient of the present invention, lactic acid bacteria, is not particularly limited, as long as it belongs to any of Type I IFN, Type II IFN, or Type III IFN, and, IFNs include preferably at least one or more types selected from the group consisting of IFN-$\alpha$, IFN-$\beta$ and IFN-$\lambda$, more preferably at least one type of IFNs is IFN-$\alpha$, further preferably at least one type of IFNs is IFN-$\alpha$, and, is at least one or more types selected from the group consisting of IFN-$\alpha$, IFN-$\beta$ and IFN-$\lambda$, even more preferably at least one type of IFNs is IFN-$\alpha$ and two or more types selected from the group consisting of IFN-$\alpha$, IFN-$\beta$, and IFN-$\lambda$, even more preferably at least two types of the two or more types of IFNs are IFN-$\alpha$ and IFN-$\beta$, and particularly preferably IFNs include three types, IFN-$\alpha$, IFN-$\beta$, and IFN-$\lambda$.
The fact that the active ingredient of the present invention, lactic acid bacteria, can directly activate pDCs and/or induce the production of at least one or more types of IFN can be confirmed, for example, by measuring the amount or concentration of IFN such as IFN-$\alpha$ or IFN-$\beta$ produced in a culture system as a result of the activation of pDCs when the lactic acid bacteria are cultured under the coexistence of pDCs induced from bone marrow cells of a mammal such as mice.
Specifically, this can be confirmed by measuring the IFN concentration using the following procedures (i) to (iv).

(i) Mouse-derived bone marrow cells from which red blood cells have been removed are suspended in RPMI medium prepared to have the following composition at a concentration of $1 \times 10^6$ cells/mL to prepare a cell suspension.
<Medium composition>

- 10% by volume FBS
- 100U/mL penicillin/streptomycin
- 1mM Sodium pyruvate
- 2.5 mM HEPES
- 1% by mass MEM nonessential amino acid solution (100 ×)
- 50 μM β-mercaptoethanol
- 100 ng/mL Flt-3L

(ii) 1 mL each of the prepared cell suspension is seeded and cultured in a $CO_2$ incubator at 37°C and 5% by volume of $CO_2$ for one week to induce pDCs.

(iii) Bone marrow cells containing induced pDCs are suspended at $2 \times 10^5$ cells/mL and 200 μL each of the resultant is seeded onto a 96-well plate, and then 2 μL each of the lactic acid bacteria suspension adjusted to have a concentration of 1 mg/mL with PBS is added.

(iv) 24 hours later, the culture supernatant is collected and the IFN-α concentration is measured by ELISA using an IFN-α measurement kit.

The active ingredient of the present invention, lactic acid bacteria, which can be represented by an index of the production of IFN-α at 30 pg/mL or more (preferably 50 pg/mL, more preferably 60 pg/mL, more preferably 70 pg/mL, 80 pg/mL, 90 pg/mL, 100 pg/mL, 150 pg/mL, even more preferably 200 pg/mL, 210 pg/mL, 220 pg/mL, 230 pg/mL, 240 pg/mL, 250 pg/mL, even more preferably 300 pg/mL, 400 pg/mL, 500 pg/mL, 600 pg/mL, 700 pg/mL, and particularly preferably 800 pg/mL), can be prepared by co-culturing the lactic acid bacteria having a final concentration of 10 μg/mL with pDCs-containing bone marrow cells having a final concentration of $2 \times 10^5$ cells/mL for 24 hours, wherein the bone marrow cells are obtained by collecting them from mouse bone marrow followed by 7 days of culture in a 100 ng/mL of Flt3-L-containing cell culture medium.

The active ingredient of the present invention, lactic acid bacteria, are not particularly limited, but examples thereof include lactic acid bacteria belonging to the genus *Oenococcus,* the genus *Bifidobacterium,* the genus *Lentilactobacillus,* the genus *Weissella,* the genus *Tetragenococcus,* the genus *Lactococcus,* the genus *Leuconostoc,* the genus *Pediococcus,* the genus *Streptococcus,* the genus *Enterococcus,* the genus *Lactobacillus,* and the genus *Bacillus.*

In addition, examples of the bacteria of the genus *Lactobacillus* in the present invention include bacteria that were classified into the genus *Lactobacillus* before the reclassification of the genus *Lactobacillus.* Examples thereof include bacteria newly classified after the reclassification of the genus *Lactobacillus,* such as the genus *Acetilactobacillus,* the genus *Agrilactobacillus,* the genus *Amylolactobacillus,* the genus *Apilactobacillus,* the genus *Bombilactobacillus,* the genus *Companilactobacillus,* the genus *Dellaglioa,* the genus *Fructilactobacillus,* the genus *Furfurilactobacillus,* the genus *Holzapfelia,* the genus *Lacticaseibacillus,* the genus *Lactiplantibacillus,* the genus *Lapidilactobacillus,* the genus *Latilactobacillus,* the genus *Lentilactobacillus,* the genus *Levilactobacillus,* the genus *Ligilactobacillus,* the genus *Limosilactobacillus,* the genus *Liquorilactobacillus,* the genus *Loigolactobacillus,* the genus *Paralactobacillus,* the genus *Paucilactobacillus,* the genus *Schleiferilactobacillus,* and the genus *Secundilactobacillus.*

Examples of the above bacteria of the genus *Oenococcus* include *Oenococcus oeni.* Specific examples of the bacteria of the genus *Oenococcus* include *Oenococcus oeni* JCM6125.

Examples of the above bacteria of the genus *Bifidobacterium* include *Bifidobacterium animalis* subsp. *lactis* and *Bifidobacterium longum* subsp. *infantis.* Specific examples of the bacteria of the genus *Bifidobacterium* include *Bifidobacterium animalis* subsp. *lactis* JCM10602, *Bifidobacterium longum* subsp. *infantis* JCM1222, and *Bifidobacterium longum* subsp. *longum* BB536.

Examples of the above bacteria of the genus *Weissella* include *Weissella paramesenteroides* and *Weissella viridescens.* Specific examples of the bacteria of the genus *Weissella* include *Weissella paramesenteroides* JCM9890 and *Weissella viridescens* JCM1174.

Examples of the above bacteria of the genus *Tetragenococcus* include *Tetragenococcus halophilus.* Specific examples of the bacteria of the genus *Tetragenococcus* include *Tetragenococcus halophilus* NRIC0098 and *Tetragenococcus halophilus* No. 1.

Examples of the bacteria of the genus *Lactococcus* include *Lactococcus lactis, Lactococcus lactis* subsp. *lactis, Lactococcus garvieae, Lactococcus lactis* subsp. *cremoris, Lactococcus lactis* subsp. *hordniae,* and *Lactococcus plantarum.*

Specific examples of the above bacteria of the genus *Lactococcus* include *Lactococcus lactis* subsp. *lactis* JCM5805, *Lactococcus lactis* subsp. *lactis* NBRC12007, *Lactococcus lactis* subsp. *lactis* NRIC1150, *Lactococcus lactis* subsp. *lactis* JCM20101, *Lactococcus lactis* subsp. *lactis* JCM7638, *Lactococcus lactis* subsp. *lactis* ATCC 7963, *Lactococcus lactis* subsp. *lactis* ATCC 7962, *Lactococcus lactis* subsp. *lactis* ATCC 29146, *Lactococcus lactis* subsp. *lactis* ATCC 27861, *Lactococcus lactis* subsp. *lactis* ATCC 19435, *Lactococcus lactis* subsp. *lactis* ATCC 15577, *Lactococcus lactis* subsp. *lactis* ATCC 15346, *Lactococcus lactis* subsp. *lactis* ATCC 13675, *Lactococcus lactis* subsp.

*lactis* ATCC 12929, *Lactococcus lactis* subsp. *lactis* ATCC 11955, *Lactococcus lactis* subsp. *lactis* ATCC 11454, *Lactococcus lactis* subsp. *lactis* ATCC11007, *Lactococcus garvieae* NBRC100934, *Lactococcus lactis* subsp. *cremoris* JCM16167, *Lactococcus lactis* subsp. *cremoris* NBRC100676, *Lactococcus lactis* subsp. *holdoniae* JCM1180, and *Lactococcus lactis* subsp. *holdoniae* JCM11040, and *Lactococcus plantarum* JCM11056.

Examples of the above bacteria of the genus *Leuconostoc* include *Leuconostoc carnosum* and *Leuconostoc lactis.* Specific examples of the bacteria of the genus *Leuconostoc* include *Leuconostoc carnosum* JCM9695 and *Leuconostoc lactis* NBRC12455.

Examples of the bacteria of the genus *Pediococcus* include *Pediococcus acidilactici, Pediococcus pentosaceus, Pediococcus cellicola, Pediococcus claussenii, Pediococcus damnosus, Pediococcus ethanolidurans, Pediococcus inopinatus, Pediococcus parvulus,* and *Pediococcus stilesii.* Specific examples of the bacteria of the genus *Pediococcus* include *Pediococcus acidilactici* JCM8797, *Pediococcus acidilactici* K15, and *Pediococcus damnosus* JCM5886.

An example of the bacteria of the genus *Streptococcus* is *Streptococcus thermophilus.* A specific example of the bacteria of the genus *Pediococcus* is *Streptococcus thermophilus* SBC8781.

Examples of the bacteria of the genus *Enterococcus* include *Enterococcus alcedinis* and *Enterococcus faecalis.* Specific examples of the bacteria of the genus *Enterococcus* include *Enterococcus faecalis* EC-12.

Examples of the bacteria of the genus *Lactobacillus* include *Lactobacillus paracasei, Lactobacillus delbrueckii, Lactobacillus acidophilus, Lactobacillus casei, Lactobacillus fructivorans, Lactobacillus hilgardii, Lactobacillus rhamnosus, Lactobacillus gasseri, Lactobacillus acidophilus, Lactobacillus bulgaricus, Lactobacillus parakefiri, Lactobacillus plantarum,* and *Lactobacillus pentosus.*

Specific examples of the bacteria of the genus *Lactobacillus* include *Lactobacillus paracasei* KW3110, *Lactobacillus paracasei* MCC1849, *Lactobacillus paracasei* K71, *Lactobacillus paracasei* K-2, *Lactobacillus rhamnosus* GG, *Lactobacillus rhamnosus* CRL1505, *Lactobacillus gasseri* SBT2055, *Lactobacillus acidophilus* L-92, *Lactobacillus casei* subsp. *casei 327, Lactobacillus* (newly classified as *Lacticaseibacillus) casei Shirota, Lactobacillus parakehfilii* (newly classified as *Lentilactobacillus parakehfilii)* JCM8573, *Lactobacillus plantarum* (newly classified as *Lactiplantibacillus plantarum)* L-137, and *Lactobacillus pentosus* (newly classified as *Lactiplantibacillus pentosus)* ONRICb0240.

Examples of the above bacteria of the genus *Bacillus* include, but are not particularly limited to, *Bacillus coagulans.* Specific examples of the bacteria of the genus *Bacillus* include *Bacillus coagulans* SANK70258 strain.

Among the active ingredient of the present invention, for example, lactic acid bacteria, the JCM strain can be obtained from the Microbe Division/Japan Collection of Microorganisms RIKEN BioResource Research Center (3-1-1 Koyadai, Tsukuba, Ibaraki 305-0074, Japan), the NBRC strain can be obtained from the Biological Resource Center, National Institute of Technology and Evaluation (5-8 Kazusa Kamatari 2-chome, Kisarazu, Chiba Prefecture, Japan), the NRIC strain can be obtained from the Center for Microorganism Resources (Formerly, Culture collection center), Tokyo University of Agriculture (1-1 Sakuragaoka 1-chome, Setagaya-ku, Tokyo, Japan), and the ATCC strain can be obtained from the American Type Culture Collection (USA). In addition to obtainment from public institutions, the active ingredient of the present invention, lactic acid bacteria, can be obtained by isolating or purifying the lactic acid bacteria from commercially available products that contain them using known methods.

The active ingredient of the present invention, lactic acid bacteria, may be a culture of lactic acid bacteria. Examples of the culture include live bacteria, dead bacteria, crushed live or dead bacteria, freeze-dried live or dead bacteria, crushed freeze-dried products, enzyme-treated live or dead bacteria, and culture liquids or culture liquid extracts, and further include portions of lactic acid bacteria and processed products of lactic acid bacteria. Dead bacteria can be obtained from live bacteria by, for example, heat treatment, treatment with drugs such as antibiotics, treatment with chemicals such as formalin, treatment with ultraviolet rays, or treatment by radiation such as gamma rays. Examples of processed products include heated bacteria (dead bacteria), freeze-dried products thereof, and cultures containing them, and further include liquids in which bacteria are crushed by ultrasound or the like, and liquids in which bacteria are treated with enzymes. Examples of the processed products also include processed products in which cell walls have been removed by enzymes or mechanical means. Furthermore, nucleic acid-containing fractions obtained by lysing bacteria with a surfactant or the like and then precipitating with ethanol or the like are also included. Examples of the active ingredient of the present invention, lactic acid bacteria; that is, a culture of the lactic acid bacteria, also include DNA or RNA of the lactic acid bacteria. The DNA or RNA of the lactic acid bacteria can activate pDCs, so as to induce the production of IFN.

Lactic acid bacteria can be cultured by a known method using a known medium. As the medium, MRS medium, GAM medium, or LM17 medium can be used, and inorganic salts, vitamins, amino acids, antibiotics, and/or serum, etc., can be added and used as appropriate. Culturing may be carried out at 25°C to 40°C for several hours to several days.

After culturing, the lactic acid bacteria cells are collected from the resulting culture liquid of the lactic acid bacteria by centrifugation or filtration, etc., so that the lactic acid bacteria of interest can be obtained. When using the lactic acid bacteria as dead bacteria, they may be sterilized and inactivated by autoclaving, etc.

It is preferable that the composition of the present invention is used via oral intake in view of reducing the burden of intake. When used via oral intake, it is preferable that the lactic acid bacteria or a composition containing the lactic acid bacteria has high resistance to gastric fluids and intestinal fluids, for example, strong acid resistance. There are no particular limitations on the lactic acid bacteria, and both live and dead bacteria can be used, but it is preferable that the lactic acid bacteria are dead bacteria in view of immunostimulatory effect, stability, production efficiency, etc., and it is more preferable that the lactic acid bacteria are heated dead bacteria.

The composition of the present invention can be provided in the form of a composition in which lactic acid bacteria are mixed with other ingredients (e.g., food raw materials, food additives, formulation additives). The content of lactic acid bacteria in the composition can be determined based on the manner in which the composition is provided and the effective intake described below, and the lower limit (or more or exceeding) can be, for example, 0.01% by mass, 0.1% by mass, 1% by mass, 10% by mass, or 20% by mass, and the upper limit (or more or less than) can be, for example, 100% by mass, 90% by mass, 80% by mass, 70% by mass, 60% by mass, or 50% by mass. These lower and upper limits can be arbitrarily combined, and the content of lactic acid bacteria in the composition can be, for example, 0.01% to 100% by mass, 0.1% to 90% by mass, 10% to 80% by mass, or 20% to 70% by mass. Further, the content of lactic acid bacteria in the composition of the present invention can be determined by the number of lactic acid bacteria, and the lower limit (or more or exceeding) can be, for example, $1 \times 10^8$ cells, $1 \times 10^9$ cells, or $1 \times 10^{10}$ cells, and the upper limit is not particularly limited, and can be, for example, $1 \times 10^{14}$ cells, $1 \times 10^{13}$ cells, $1 \times 10^{12}$ cells, $4 \times 10^{11}$ cells, or $1 \times 10^{11}$ cells. These upper and lower limits can be arbitrarily combined, and the range of the intake can be, for example, $1 \times 10^8$ cells to $1 \times 10^{14}$ cells, $1 \times 10^9$ cells to $1 \times 10^{13}$ cells, $1 \times 10^{10}$ cells to $1 \times 10^{12}$ cells, $1 \times 10^9$ cells to $4 \times 10^{11}$ cells, or $1 \times 10^9$ cells to $1 \times 10^{11}$ cells.

The composition of the present invention can be used for the purpose of preventing or treating chemosensory disorders. Here, the chemosensory disorders refer to disorders of the senses caused by chemical stimuli, and preferably refer to chemosensory disorders that occur after viral infection. When the chemosensory disorders are disorders that occur due to viral infection, they can be chemosensory disorders that occur within 7 days after viral infection. The chemosensory disorders can also be particularly disorders of taste or olfaction, among the five senses. In the present invention, "prevention" is used in a sense including the ease of symptoms at the onset of chemosensory disorders and reducing the risk of developing chemosensory disorders. In the present invention, "treatment" is used in a sense including the improvement of the symptoms of chemosensory disorders and reducing the risk of increased severity of chemosensory disorders. The composition of the present invention can be provided as a preventive or therapeutic agent for chemosensory disorders, which contains lactic acid bacteria as an active ingredient.

Examples of viruses that cause chemosensory disorders include SARS-CoV-2 (novel coronavirus), rhinovirus, coronavirus, parainfluenza virus, and Epstein-Barr virus. Here, SARS-CoV-2 includes not only the virus strain that was first discovered, but also its mutant strains (e.g., B.1.1.7 lineage (alpha strain), B.1.351 lineage (beta strain), P.1 lineage (gamma strain), B.1.617.2 lineage (delta strain), B.1.1.529 lineage (omicron strain)), and JN. 1 lineage. SARS-CoV-2 is synonymous with severe acute respiratory syndrome coronavirus-2, and SARS-CoV-2 infection (diseases and symptoms caused by infection with SARS-CoV-2) is called COVID-19. The composition of the present invention can preferably be used for the purpose of preventing or treating COVID-19 sequelae.

The composition of the present invention is used for preventing or treating chemosensory disorders. For example, when the chemosensory disorder is a taste disorder, the preventive or therapeutic effect of the composition of the present invention on the taste disorder can be evaluated based on a questionnaire regarding subjective symptoms. Specifically, it can be evaluated that subjects not having any taste disorder have been prevented from developing taste disorders, if the incidence of a taste disorder is reduced in a group of subjects taking the composition of the present invention compared to a non-taking group, or if symptoms at the time of onset are eased. Moreover, it can be evaluated that subjects having taste disorders have been treated, if the proportion of subjects having taste disorders is reduced in a group of subjects taking the composition of the present invention compared to a non-taking group, or if symptoms are improved.

Furthermore, for example, when the chemosensory disorder is an olfactory disorder, the preventive or therapeutic effect of the composition of the present invention on the olfactory disorder can be evaluated based on a questionnaire regarding subjective symptoms. Specifically, it can be evaluated that subjects not having any olfactory disorder have been prevented from developing olfactory disorders, if the incidence of an olfactory disorder is reduced in a group of subjects taking the composition of the present invention compared to a non-taking group, or if symptoms at the time of onset are eased. Moreover, it can be evaluated that subjects having olfactory disorders have been treated, if the proportion of subjects having olfactory disorders is reduced in a group of subjects taking the composition of the present invention compared to a non-taking group, or if symptoms are improved.

The composition of the present invention can be provided in the form of, for example, a food composition, a pharmaceutical composition, a quasi-drug, a feed (including pet food), or an additive, and can be provided according to the following description.

The composition of the present invention can be orally taken by humans and non-human mammals, and a typical form of intake is a food composition. When the composition of the present invention is provided as a food composition, it may be provided as a food as it is, or may be provided by being contained in a food. The food provided is a food containing an effective amount of the composition of the present invention. Here, "containing an effective amount" refers to a content such that the active ingredient of the present invention is taken to an extent that the effect of immunostimulation, etc., is exerted when the amount normally consumed in each food is taken. Further, "food(s)" is used in a sense including health foods, functional foods, nutritional supplements, health functional foods (e.g., foods for specified health uses, nutritional functional foods, foods with functional claims), special purpose foods (e.g., foods for infants, foods for pregnant women, foods for sick people) and supplements. It goes without saying that when the active ingredient of the present invention is taken by mammals other than humans, the food referred to in the present invention is used as feed.

The composition of the present invention can be provided by being contained in a food that is consumed daily. In this case, the composition of the present invention can be provided in a unit package form in which the amount to be taken per meal is predetermined. Examples of a unit package form per meal include forms, in which a fixed amount is specified, such as a pack, a wrapping, a can, and a bottle. In order to better exert the various effects of the composition of the present invention, an intake per meal can be determined according to the daily intake of the active ingredient of the present invention, which will be described later. The food of the present invention may be provided with an explanation regarding the intake indicated on the package, or together with a document or the like containing the explanation.

The predetermined intake per meal in a unit package form may be the effective intake per day, or the effective intake per day divided into two or more portions (preferably 2 to 6 portions). Thus, the unit package form of the composition of the present invention can contain the active ingredient of the present invention in an amount of the daily intake described below, or can contain the active ingredient of the present invention in an amount of half or less (preferably half to one-sixth) of the daily intake described below. For the convenience of intake, it is preferable that the composition of the present invention is provided in a unit package form per meal (i.e., a unit package form per day) in which the intake per meal is the effective intake per day.

The form of the "food" is not particularly limited, and may be, for example, a beverage, a semi-liquid or a gel form, or a solid or a powder form. Examples of "supplement" include tablets produced by kneading the active ingredient of the present invention with an excipient, a binder, etc., and then tableting, granules produced by adding an excipient, a binder, etc., to the active ingredient of the present invention and granulating the resultant, orally disintegrating tablets, and capsules in which the active ingredient of the present invention is encapsulated in a capsule or the like. When provided as a supplement, in addition to the above unit packaging form per meal or per day, it is also suitable to provide it in a unit packaging form per week, per two weeks, per month, or per two months. In addition, it is preferable that the latter unit packaging form has, for example, a label on which the intake per meal or per day is described, so that the consumer can take an effective amount of the active ingredient of the present invention by following the label.

Examples of the foods provided by the present invention include, but are not particularly limited to, as long as they contain the active ingredient of the present invention: non-alcoholic beverages such as soft drinks, carbonated drinks, drinks containing fruit juice, drinks containing vegetable juice, drinks containing fruit juice and vegetable juice, livestock milk such as cow's milk, soy milk, dairy drinks, drink-type yogurt, drink-type or stick-type jellies, coffee, cocoa, tea beverages, nutritional drinks, energy drinks, sports drinks, mineral water (including both sparkling and non-sparkling), near-water, and non-alcoholic beer-flavored drinks; carbohydrate-containing foods and drinks such as rice, noodles, bread, or pasta; dairy products such as cheeses, hard type or soft type yogurt, fresh cream made from livestock milk or other fats and oils as raw materials, and ice cream; various sweets such as Western confectioneries, e.g., cookies, cakes, and chocolates, Japanese confectioneries e.g., a sweet (bean-jam) bun and a sweet jelly made from bean jam, tablet confectioneries (refreshing confectionery) e.g., *ramune* candies, candies, chewing gum, gummy candies, cold confectioneries and frozen confectioneries e.g., jelly and pudding, and snacks; alcoholic beverages such as whiskey, bourbon, spirits, liqueur, wine, fruit wine, sake, Chinese alcohol, shochu, beer, non-alcoholic beer with an alcohol content of 1% or less, sparkling wine, other miscellaneous alcohols, and cocktail of Japanese spirits; processed foods such as processed foods using eggs, processed seafood or meat (containing other organ meats such as liver) (including delicacies), and soups such as miso soup; condiments such as miso, soy sauce, *furikake* (rice seasoning), and other seasonings; or liquid foods such as thick liquid foods.

Examples of tea beverages include fermented, semi-fermented and non-fermented teas, such as black tea, green tea, barley tea, brown rice tea, *sencha, gyokuro* (refined green) tea, roasted green tea, oolong tea, turmeric tea, pu-erh tea, rooibos tea, rose tea, chrysanthemum tea, ginkgo leaf tea and herbal teas (e.g. mint tea and jasmine tea).

Examples of fruits to be used in fruit juice-containing drinks and drinks containing fruit juice and vegetable juice include apples, mandarin oranges, grapes, bananas, pears, peaches, mangoes, acai, blueberries, and plums. Examples of vegetables to be used in vegetable juice-containing drinks and drinks containing fruit juice and vegetable juice include tomatoes, carrots, celery, pumpkin, cucumber, and watermelon.

When the composition of the present invention is provided as feed, this can be performed according to the description for the above foods.

When the composition of the present invention is provided as a pharmaceutical composition such as a pharmaceutical product or quasi-drug, it can be formulated into an oral or parenteral agent and then the agent can be provided. Examples of oral agents include granules, powders, tablets (including sugar-coated tablets), pills, capsules, syrups, liquids, jellies, emulsions, and suspensions. Examples of parenteral agents include injections suitable for local administration (including intradermal, subcutaneous, intramuscular, and intravenous injections), inhalants (e.g., inhalation aerosols, inhalation powders, and inhalation liquids), nasal drops (e.g., nasal powders and nasal liquids), ointments, creams, gels, suppositories, patches, and poultices. These preparations can be formulated using pharmaceutically acceptable carriers by techniques commonly used in the art. Examples of pharmaceutically acceptable carriers include excipients, binders, diluents, additives, flavors, buffers, thickeners, colorants, stabilizers, emulsifiers, dispersants, suspending agents, and preservatives.

When the composition of the present invention is provided as an additive, this can be performed according to the above description concerning foods, feed, pharmaceutical products, quasi-drugs or pharmaceutical compositions. When the composition of the present invention is provided as a food additive, the composition of the present invention can be used as a functionally active ingredient in foods for specified health use or a food with functional claims having an effect of preventing or treating chemosensory disorders.

The composition of the present invention may be labeled with a label indicating that it has an effect of improving or easing chemosensory disorders. In this case, some or all of the following labels may be attached to the composition of the present invention in order to make the labeling easy for consumers to understand.

- For those concerned about chemosensory disorders (particularly olfaction and taste)
- For those concerned about taste disorders
- For those concerned about olfactory disorders
- For those concerned about olfactory and taste disorders
- For those concerned about a decline in taste in everyday life
- For those concerned about a decline in smell in everyday life
- Helps maintain taste function
- Helps maintain olfactory function
- Helps maintain olfactory and taste functions
- Suppresses the decline in taste function
- Suppresses the decline in olfactory function
- Suppresses the decline in olfactory and taste functions

The composition of the present invention is intended to be used exclusively in therapeutic forms, but some forms of applications may also include non-therapeutic applications. Non-limiting examples of non-therapeutic applications include applications for use under labels that are easy for consumers to understand, as described above, and applications for use in a form to be taken by healthy individuals.

The intake of the composition of the present invention can be determined depending on the recipient's sex, age, and body weight, symptoms, intake time, dosage form, intake route, and materials or drugs, etc., to be combined, etc. The daily intake of the composition of the present invention for an adult can be specified, for example, by the number of, the active ingredient, lactic acid bacteria, and the lower limit is not particularly limited, but can be, for example, $1 \times 10^8$ cells, $1 \times 10^9$ cells, or $1 \times 10^{10}$ cells, and the upper limit is not particularly limited, but can be, for example, $1 \times 10^{14}$ cells, $1 \times 10^{13}$ cells, $1 \times 10^{12}$ cells, $4 \times 10^{11}$ cells, or $1 \times 10^{11}$ cells. These upper and lower limits can be combined arbitrarily, and the range of the intake can be, for example, $1 \times 10^8$ cells to $1 \times 10^{14}$ cells, $1 \times 10^9$ cells to $1 \times 10^{13}$ cells, $1 \times 10^{10}$ cells to $1 \times 10^{12}$ cells, $1 \times 10^9$ cells to $4 \times 10^{11}$ cells, or $1 \times 10^9$ cells to $1 \times 10^{11}$ cells. The number of the lactic acid bacteria can be measured using a known microscope, a flow cytometer, or a non-culture-based microbiological rapid testing device (e.g., ELESTA PixeeMo (AFI Technology Co., Ltd.)), or the like, but measurement using a microscope is preferred from the viewpoint of high versatility. In the present invention, "intake" and "administration" are used interchangeably.

The adult daily intake of the composition of the present invention can also be specified based on the dried cell mass of the lactic acid bacteria, the active ingredient, and the lower limit is not particularly limited and can be, for example, $2.5 \times 10^{-2}$ mg, $2.5 \times 10^{-1}$ mg, 2.5 mg, 10 mg, 20 mg, 30 mg or 50 mg, and the upper limit is not particularly limited, and can be, for example, $2.5 \times 10^4$ mg, $2.5 \times 10^3$ mg, $2.0 \times 10^3$ mg, or $2.5 \times 10^2$ mg. These upper limit and lower limit can be arbitrarily combined, and the range of the intake can be, for example, $2.5 \times 10^{-2}$ mg to $2.5 \times 10^4$ mg, $2.5 \times 10^{-1}$ mg to $2.5 \times 10^3$ mg, 2.5 mg to $2.5 \times 10^2$ mg, or 20 mg to $2.0 \times 10^3$ mg.

The composition of the present invention can be administered to a subject in need of prevention or treatment of a chemosensory disorder. Subjects in need of chemosensory disorder prevention or treatment are not particularly limited, but examples thereof include subjects who have chemosensory disorders, subjects who are infected with a virus, subjects who have chemosensory disorders due to viral infection, subjects who have a cold, and subjects who have chemosensory disorders due to a cold. The above virus is preferably SARS-CoV-2. Non-limiting examples of SARS-CoV-2 positive subjects include asymptomatic or mildly symptomatic SARS-CoV-2 positive subjects, specifically SARS-CoV-2 positive subjects without respiratory symptoms or with only a cough and no breathing difficulties.

Targets for intake or administration of the composition of the present invention include mammals, including humans. Examples of mammals are not particularly limited to, but include, primates, rodents, ungulates, and carnivores, with primates being preferred. Non-limiting examples of mammals include humans, monkeys, dogs, cats, cows, horses, pigs, and sheep, with humans being preferred.

It is preferable that the composition of the present invention is taken continuously for a period during which the preventive or therapeutic effect on the chemosensory disorder is expected. From the viewpoint of better exerting the preventive or therapeutic effect on the chemosensory disorder, the period of taking the daily dose of the active ingredient of the present invention can be, for example, one or more weeks, two or more weeks, three or more weeks, and preferably one or more months (four weeks or more). The interval of taking the active ingredient of the present invention can be once every three days, once every two days, or once a day at the above daily dose, and is preferably once a day.

The intake of the composition of the present invention may also be started before an event or time when a preventive or therapeutic effect on a chemosensory disorder is expected. Examples of events, for which a preventive or therapeutic effect on a chemosensory disorder is expected, include actions that may result in viral infection (e.g., participation in an event with a high risk of viral infection, traveling to an epidemic area), and examples of times when a preventive or therapeutic effect on a chemosensory disorder is expected include a viral infection epidemic period. Examples of the timing of intake before an event, for which a preventive or therapeutic effect on a chemosensory disorder is expected, include one or more days before, three or more days before, one or more weeks before, two or more weeks before, three or more weeks before, one or more months before (four or more weeks before), or two or more months before (eight or more weeks before). In addition, although not particularly limited, the composition can also be taken continuously with an interval between intakes from the start of intake until an event, for which a preventive or therapeutic effect on a chemosensory disorder is expected, in some cases. The intake of the active ingredient of the present invention may also be started after an event or time when a preventive or therapeutic effect on a chemosensory disorder is expected. Examples of the timing of intake after an event, for which a preventive or therapeutic effect on a chemosensory disorder is expected, include one or more days later, three or more days later, one or more weeks later, or two or more weeks later. In addition, although not particularly limited, when intake is performed after an event, for which a preventive or therapeutic effect on a chemosensory disorder is expected, in some cases, intake can be continued with intervals between intakes. In the present invention, particularly preferably the intake of the active ingredient of the present invention can be started before an event, for which a preventive or therapeutic effect on a chemosensory disorder is expected, and then continued until after the event.

According to another aspect of the present invention, a method for preventing or treating a chemosensory disorder, comprising causing a subject in need thereof to take or administering to a subject in need thereof an effective amount of a lactic acid bacterium or a composition containing the same. The method of the present invention can be performed according to the description concerning the composition of the present invention.

According to another aspect of the present invention, the use of lactic acid bacteria for the manufacture of a composition for preventing or treating a chemosensory disorder, the use of the lactic acid bacteria in a method for preventing or treating a chemosensory disorder, the use of the lactic acid bacteria for preventing or treating a chemosensory disorder, or, the use of the lactic acid bacteria as a composition for preventing or treating a chemosensory disorder is provided. According to the present invention, the use of a composition comprising lactic acid bacteria in a method for preventing or treating a chemosensory disorder, the use of a composition comprising lactic acid bacteria for preventing or treating a chemosensory disorder, or the use of a composition comprising lactic acid bacteria as a composition for preventing or treating a chemosensory disorder is also provided. The use of the present invention can be carried out according to the description concerning the composition of the present invention or the method of the present invention.

According to yet another aspect of the present invention, lactic acid bacteria for use in the prevention or treatment of a chemosensory disorder is provided. According to the present invention, a composition comprising the lactic acid bacteria for use in the prevention or treatment of a chemosensory disorder is also provided. The above lactic acid bacteria can be used according to the description concerning the composition of the present invention.

**Examples**

The present invention will be described in more detail with reference to the following examples, but the present invention is not limited to these examples.

Verification of the effect of *L. lactis* strain Plasma in preventing the onset of and easing the symptoms in SARS-CoV-2 positive individuals

1. Overview

This study relates to the verification (PLATEAU study) of the efficacy of *Lactococcus lactis* strain Plasma (LC-Plasma) to ease symptoms in COVID-19 patients with coronavirus disease 2019 (COVID-19) using *L. lactis* strain Plasma. The efficacy to ease the symptoms was verified by a multi-center, double-blinded, randomized placebo-controlled trial.

2.1 Significance of study and basis for scientific rationality

Plasmacytoid dendritic cells (pDCs) are known to have functions as a command center in defense against viral infections. It is known that when pDCs are activated, interferon-a is produced through the innate immune system, triggering an early response to eliminate the virus, while viral antigen-specific killer T cells and B cells are induced through the adaptive immune system, triggering a late response. *Lactococcus lactis* strain Plasma was isolated as a bacterial strain that activates pDCs[1].

In COVID-19 patients, after infection with SARS-CoV-2, in young people having rapid increases in interferon, the SARS-CoV-2 viral load decreases rapidly after exposure, whereas in elderly people having slow increases in interferon, the SARS-CoV-2 viral load decreases slowly and an inflammatory response occurs[2], suggesting that the reactivity of natural immunity early in SARS-CoV-2 infection greatly influences subsequent symptoms.

On the other hand, it has been reported[3] that healthy individuals who had taken *L. lactis* strain Plasma had improved interferon responsiveness of peripheral blood mononuclear cells (PBMCs) when exposed to inactivated influenza virus, so it is possible that taking *L. lactis* strain Plasma may also be effective against COVID-19, specifically, in preventing the onset and easing the symptoms of COVID-19 through activation and maintenance of the natural immune system.

It has also been reported[4] that cooperation between CD4[+] T cells and CD8[+] T cells, which are part of the adaptive immune system, is important in inhibiting the increased severity of COVID-19. Since the proportion of antigen-specific CD8[+] T cells increased[5] in mice when they were fed with *L. lactis* strain Plasma, it is expected that the intake of *L. lactis* strain Plasma may also be effective in easing COVID-19 symptoms through the adaptive immune system.

Based on these findings, this study was conducted for the purpose of examining the effect of *L. lactis* strain Plasma in preventing the onset of and easing the symptoms in subjects, SARS-CoV-2 positive individuals with no symptom or SARS-CoV-2 patients with mild symptoms.

2.2 Purpose of this study

This study was conducted for the purpose of examining the effect of *L. lactis* strain Plasma in preventing the onset of and easing the symptoms in SARS-CoV-2-positive individuals.

2.3 Study foods subjected to this study (intervention)

2.3.1 Overview of study foods

In this study, study subjects were required to take a determined amount of one of the following study foods every day after breakfast, in principle.

Generic name:

Capsules containing *L. lactis* strain Plasma or capsules containing placebo food

Uses and dosages:

- Group A: *L. lactis* strain Plasma intake group
  In this study, four *L. lactis* strain Plasma-containing capsules were orally taken once a day after breakfast as a rule (dosing period: 14 days).
- Group B: Placebo intake group

In this study, four placebo food-containing capsules were orally taken once a day after breakfast as a rule (dosing period: 14 days).

The raw materials and percentages of the test foods are shown in Table 1. For details of allocation, see "2.6 Allocation and maintenance of blinding."

In this example, *L. lactis* strain Plasma refers to *Lactococcus lactis* subsp. *lactis* JCM 5805. The JCM strain can be obtained from the Microbe Division/Japan Collection of Microorganisms RIKEN BioResource Research Center (3-1-1 Koyadai, Tsukuba, Ibaraki 305-0074, Japan).

[Table 1]

| Table 1:Raw materials and percentage thereof per capsule | | | | | | |
|---|---|---|---|---|---|---|
| | *L. lactis* strain Plasma-containing capsule | | | Placebo food-containing capsule | | |
| Raw material name | Bacterial count (cells) | Prescrip tion (mg) | Percentage (mass%) | Bacterial count (cells) | Prescrip tion (mg) | Percentage (mass%) |
| Lactic acid bacteria pow-der *(L. lactis* strain Plas-ma) | $1\times10^{11}$ | 50 | 27.8 | 0 | 0 | 0 |
| Paindex #100 | - | 127.3 | 70.7 | - | 177.3 | 98.5 |
| Calcium stearate (vegetal) | - | 2.7 | 1.5 | - | 2.7 | 1.5 |
| Total | $1\times10^{11}$ | 180 | 100 | 0 | 180 | 100 |

2.3.2 Precautions for use of study foods and disadvantages (anticipated side effects) due to intake thereof etc.

In a previous safety study on excessive intake, when 250 mg of *L. lactis* strain Plasma was taken per day for four weeks, which is higher than the intake in this study (200 mg, approximately 400 billion *L. lactis* strain Plasma cells), clinically problematic findings were not found by questioning, physicochemical tests, hematological tests, blood biochemistry tests, and urine tests, and no adverse events thought to be attributable to the study foods were observed[6], confirming that there are no safety issues with excessive intake of *L. lactis* strain Plasma.

Further, products containing *L. lactis* strain Plasma are sold in the form of soft drinks by Kirin Beverage Co., Ltd., yogurt by Koiwai Dairy Products Co., Ltd., and supplements by Kirin Holdings Co., Ltd. and Kyowa Hakko Bio Co., Ltd. All of these products contain over 100 billion *L. lactis* strain Plasma cells, the effective dose for antiviral infection, and have been on the market since 2012, but no health hazards have been reported to date. Based on this, it was determined that taking the intake of *L. lactis* strain Plasma in this study (200 mg, approximately 400 billion *L. lactis* strain Plasma cells) for 14 days is safe. However, it was assumed that abdominal symptoms such as abdominal pain, constipation, loose stools, and diarrhea may rarely occur as side effects when consuming common foods.

2.4 Study subjects (target patients)

The following inclusion criteria and exclusion criteria were established from the viewpoint of meeting the objectives of this study and ensuring the safety of study subjects. In addition, patients who required a legally acceptable representative were excluded in order to properly evaluate efficacy.

2.4.1 Inclusion criteria

Patients were included if they met all of the following criteria.

1. SARS-CoV-2 positive patients
2. Patients with $SpO_2$ value of 96% or higher
3. Males and females aged 20 or older and less than 65 at the time of obtaining consent
4. Patients who do not refuse to disclose whether or not they have been vaccinated
5. Patients who are able to receive medical treatment at accommodation and care facilities designated by Nagasaki Prefecture*
6. Patients whose written or electromagnetic consent to participate in this study has been obtained.

*However, patients who have been scheduled for hospitalization at the time of obtaining consent were eligible to enroll.

<Basis of setting>

1-4. These were set in order to appropriately evaluate the endpoints in this study.

5. This was set taking into consideration the feasibility of the study.

6. This was set to ensure the voluntary participation of study subjects and their safety. Participation in the study with the consent of a legally acceptable representative is not permitted.

2.4.2 Exclusion criteria

Patients who met any of the following criteria were excluded from this study.

1. Obese (BMI $\geq$ 30 kg/m$^2$) patients

2. Patients who have severe breathing difficulties, chest pain, bloody sputum

3. Patients with a history of COVID-19

4. Patients using or planning to use neutralizing antibody preparations

5. Patients who are using immunosuppressants, antirheumatic drugs, corticosteroids, immunoglobulin preparations at the time of obtaining consent

6. Patients who are taking intestinal regulators

7. Patients who consume every day one or more drinks containing *L. lactis* Plasma, yogurt (yogurt containing lactic acid bacteria, *Lactobacillus bulgaricus* or the like), lactobacillus drinks

8. Patients who are pregnant, breastfeeding, or may be pregnant

9. Patients who are participating in other interventional clinical trials at the time of obtaining consent

10. Patients who require a legally acceptable representative

11. Other patients who are deemed inappropriate to participate in this study by the primary investigator or sub-investigator

<Basis of setting>

1-2, 4, 5, 8-11. These were set taking into consideration the safety of study subjects.

3,6,7 These were set to appropriately evaluate the endpoints in this study.

2.4.3 Discontinuation criteria

If the primary investigator or sub-investigator determines that it is impossible to continue the intervention for a study subject for any of the following reasons, the primary investigator or sub-investigator will take necessary measures, such as discontinuing the intake of the study foods.

The data of the study subject were handled as "a case of study discontinuation," and the date and time of discontinuation, the reason(s) for discontinuation, and the progress were recorded in the medical record and CRF, as well as at the time of discontinuation, necessary tests were carried out and efficacy and safety were evaluated. Even if the study is discontinued, when subsequent observation is possible, observation will be continued as long as possible to conduct safety analysis, etc. In such cases, no treatment restrictions will be placed on the study subject. If observation could no longer be continued, for example, if the study subject requested to withdraw from the study or withdrew consent, such case was treated as a "dropout case."

Intervention discontinuation criteria for individual study subjects

1. If a study subject declines to participate in the study or withdraws consent

2. If a major non-conformity is discovered after enrollment

3. When it is determined that continued taking of the study food(s) is undesirable due to the worsening of the primary disease or complications

4. If it is difficult to continue the intervention due to adverse events or diseases, etc.

5. If pregnancy is discovered

6. Significantly poor adherence to the intake of study foods (when it is determined to be less than 80%, or more than 120% of the total planned intake frequency)

7. If the primary investigator or sub-investigator determines that it is appropriate to discontinue the intervention for other reasons

2.5 Study design and classification

Study design: Multicenter, randomized, double-blind, parallel group comparison, exploratory study
Study classification: Specific clinical study, Investigator-Initiated Study (IIS)

After obtaining consent, study subjects will be randomly allocated to one of the following two groups at the time of allocation and enrollment.

- Group A: *L. lactis* strain Plasma intake group (LC-Plasma)
- Group B: Placebo intake group (Placebo)

The study design is shown in Fig. 1. In this study, study subjects who had given written consent were randomly allocated to Group A (*L. lactis* strain Plasma group) or Group B (placebo group). Further, observation points are shown in Table 2.

[Table 2]

| Table 2: Observation point and schedule | | | | | | |
|---|---|---|---|---|---|---|
| Table 1 | At consent / enrollment | Observation point-1* (day 1/ baseline) | Observation point-2 (day 4 ± 1 day) | Observation point-3 (day 3 ± 1 day) | Observation point-4 (day 4 ± 1 day) | At discontinuation ** |
| (1) Eligibility information | ○ | | | | | |
| (2) Background information of subject | | ○ | | | | |
| (3) COVID-19 severity | | ○ | ○ | ○ | | △ |
| (4) Special blood test #1 | | ○ | ○ | ○ | | △ |
| (5) Special blood test #2 | | ○ | ○ | ○ | | △ |
| (6) PCR test | | ○ | ○ | ○ | | △ |
| (7) Drug-taking status | | ○ | ○ | ○ | | △ |
| (8) Vital sign | | ○ (spanning) | | | | △ |
| (9) Study food intake status | | ○ (spanning) | | | | △ |
| (10) Subjective symptoms | | ○ (spanning) | | | | △ |
| (11) Diseases etc., and adverse events | | ○ (spanning) | | | | △ |

O: Essential item △: Optional item
*Observation items or observation point-1 (day 1) can be performed simultaneously with enrollment. At this time the data to be used herein were determined to be those before the start of taking each study food.
**Upon discontinuation, information to be used were determined to be those obtained up to that time point.

2.6 Allocation and maintenance of blinding

In this study, subjects were dynamically allocated to two groups via a web system using a minimization method with the allocation factors of background factors of study subjects, age (less than 50 years old/50 or more years old), whether they had taken anti-SARS-CoV-2 viral drugs, and whether they had received the SARS-CoV-2 vaccine. In addition, the following means were taken to maintain blinding.

1) A person responsible for managing the study foods blinded the study foods provided by a person responsible for preparing the study foods for each group according to the central registration number allocation correspondence table created and provided by a person responsible for statistical analysis, and confirmed before the start of the study and before key-opening that the packaging form and labels of the study foods were indistinguishable by appearance.
2) In the event of an emergency, such as the occurrence of a serious adverse event, if a primary investigator or a sub-investigator requests key opening, the person responsible for preparing the study foods will disclose only the information about the allocated group of the case.
3) The blinding of this study was maintained for all parties involved according to the "central registration number" kept by the primary investigator or sub-investigator at the medical institution, the "central registration number allocation correspondence table" kept by the person responsible for statistical analysis, and the "study foods correspondence table" kept by the person responsible for preparing the study foods.

2.7 Concomitant therapy

During the participation period of individual study subjects in the study (from enrollment to the end of the test at observation point 4 (day 14)), concomitant use of intestinal regulators (e.g., Biofermin) was prohibited in both groups.
Moreover, as symptomatic treatment for COVID-19, regular use of cough suppressants, expectorants, general cold medicines, antipyretics (acetaminophen only), and antidiarrheals was prohibited, but occasional use for approximately three days was permitted as needed.
Furthermore, during the participation period of individual study subjects in this study, pharmaceutical products, supplements, etc., used at the time of obtaining consent were not discontinued, switched, or changed in dosage in principle, and new pharmaceutical products, supplements, etc., were not added, except for pharmaceutical products for the treatment of COVID-19 and study foods. In addition, the intake of other lactic acid bacteria-containing foods, such as yogurt similar to the study foods was discouraged.

2.8 Observation items and observation schedule

The primary investigator or the sub-investigator collected the following information (Table 3) and submitted it to the data center using CRF. The study subjects' diaries, which were recorded by the subjects themselves, were reported using a web-based electronic medium (ePRO, Electronic Patient Reported Outcomes). The occurrence of adverse events and diseases etc., were observed from time to time as an indicator of safety, regardless of the observation points in this study.

[Table 3]

| (1) Eligibility information | |
|---|---|
| Acquisition time | At enrollment |
| Information contents | inclusion criteria, exclusion criteria, date of birth (age), SARS-CoV-2 detected date, date of obtaining consent (year, month and day), allocation factor, name of medical institution/accommodation and care facility, hospitalization date/institutionalization date |

(2) Background information of study subject

| Acquisition time | At enrollment or observation point 1 (day 1) |
|---|---|
| Information contents | sex, body height, body weight, BMI*, presence or absence of the onset of COVID-19, estimated date of developing the disease, onset date in the case of onset, with or without SARS-CoV-2 vaccination, vaccination date and vaccine type when vaccinated for SARS-CoV-2, vaccination site * calculated with the formula. |

(3) COVID-19 severity

| Acquisition time | At enrollment or observation point 1 (day 1), observation point 2 (day 4), observation point 3 (day 8) |
| --- | --- |
| Information contents | Classification of severity based on the clinical management of patients with COVID-19 Version 5.0 [8] |

(4) Special blood test#1

| Acquisition time | At enrollment or observation point 1 (day 1), observation point 2 (day 4), observation point 3 (day 8) |
| --- | --- |
| Information contents | Differential white blood cell count (neutrophil, lymphocyte, eosinophil, monocyte, basophil), blood platelet, AST, ALT, $\gamma$-GTP, BUN, Cre, LDH, CRP, ferritin, HbA1c |

(5) Special blood test#2

| Acquisition time | At enrollment or observation point 1 (day 1), observation point 2 (day 4), observation point 3 (day 8) |
| --- | --- |
| Information contents | - pDCs activation markers (HLA-DR, CD86)<br>- Blood cytokines (IL-6, MCP-1)<br>- SARS-CoV-2 specific antibody level (IgM, IgG)<br>- Interferon and interferon inducible antiviral factor expression levels<br>- Immune cell analysis (T-cell, B-cell) |

(6) PCR test

| Acquisition time | At enrollment or observation point 1 (day 1), observation point 2 (day 4), observation point 3 (day 8) |
| --- | --- |
| Information contents | SARS-CoV-2 viral load (measured by PCR method using nasopharyngeal swab) |

(7) Drug-taking status

| Acquisition time | At enrollment or observation point 1 (day 1), observation point 2 (day 4), observation point 3 (day 8) |
| --- | --- |
| Information contents | Presence or absence and type of drug used during the study period. |

(8) Vital sign

| Acquisition time | Observation point 1 (day 1) to observation point 3 (day 8) |
| --- | --- |
| Information contents | Measure and record body temperature, pulse (beats/min), $SpO_2$, respiratory rate(breaths/min) every day. |

(continued)

| Measurement cost, Measurement method | Study subject recorded the information in his or her diary (ePRO) and submitted it to the data center. |

(9) Study food intake status

| Acquisition time | Time of starting the intake of study food to observation point 4 (day 14) |
| Information contents | With or without the intake of study food |
| Measurement cost, Measurement method | Study subject recorded the information in his or her diary (ePRO) and submitted it to the data center. |

(10) Subjective symptoms

| Acquisition time | Observation point 1 (day 1) to observation point 4 (day 14) |
| Information contents | Evaluate subjective symptoms (cough, breathing difficulties, fatigue, headache, olfactory or taste disorder, anorexia, and chest pain) with Severity score (4-level rating: not affected, almost not affected, affected, and severely affected) and visual analogue scale (VAS). |
| Measurement cost, Measurement method | Study subject recorded the information in his or her diary (ePRO) and submitted it to the data center. |

(11) Diseases etc., and adverse events

| Acquisition time | During observation period for relevant study subject (at enrollment to observation point 4 (day 14)) |
| Information contents | Outpatient department visit etc., with or without hospitalization, visit date or hospitalization date for treatment of COVID-19<br>Observe at any time the development of diseases etc., and the occurrence of adverse events during the observation period for relevant study subject after the start of taking the study food.<br>Diseases etc., and adverse events were determined to include various abnormal laboratory values in addition to agent side effects.<br>Information including adverse event name, time of occurrence, severity, seriousness, outcome, date of confirming outcome, association with this study, reason of the determination, drug considered to be suspected medicinal product, treatment with suspected medicinal product and other treatment contents, and drugs used, etc., was acquired principally until the end of observation. |

2.9 Evaluation of Efficacy

2.9.1 Primary endpoint

Changes in subjective symptoms* *

**Subjective symptoms (cough, breathing difficulties, fatigue, headache, olfactory or taste disorder, anorexia, chest pain) were evaluated using Severity Score (four-level rating: not affected, almost not affected, affected, and severely affected)[7] and Visual Analog Scale (VAS).

2.9.2 Secondary endpoints

1. Amounts of change or rates of change in SARS-CoV-2 viral load
2. Amounts of change or rates of change in the proportion and absolute number of pDCs, and activation markers (HLA-DR, CD86)
3. Amounts of change or rates of change in the levels of SARS-CoV-2 specific antibodies (IgM, IgG)
4. Amounts of change or rates of change in blood cytokines (IL-6, MCP-1)
5. Amounts of change or rates of change in interferon and interferon-induced antiviral factors in PBMCs
6. Outpatient visit rate and hospitalization rate

2.10 Safety evaluation

1. Amounts of change in vital signs (temperature, pulse, $SpO_2$, respiratory rate)
2. Amounts of change in blood test items
3. Incidence of adverse events such as diseases

2.11 Planned number of cases to be enrolled Target number of cases to be enrolled: 100 cases (50 subjects x 2 groups)

<Basis for setting>

The primary endpoint of this study, "changes in subjective symptoms," will be evaluated using the Severity Score (4-level rating: not affected, almost not affected, affected, and severely affected)[7]. Results were scored based on not affected: 0 point, almost not affected: 1 point, affected: 2 points, and severely affected: 3 points, and then the amount of change in the total score of 7 subjective symptoms was calculated.

In reference[7] wherein famotidine, a histamine H2 receptor antagonist, was administered to symptomatic COVID-19 patients and then their subjective symptoms were evaluated using Severity Score, the total score of Severity Scores of 10 patients listed were calculated using the calculation method in this study to give 11 ± 5.3 and 1.7 ± 2.3 on day 0 and day 14, respectively, with the amount of change of -9.3 ± 6.0.

Since this study included asymptomatic or mildly symptomatic SARS-CoV-2 positive patients with no respiratory symptoms, or, with only cough and no breathing difficulties as subjects, it was assumed that Severity Score of only cough would be 0 to 1 at observation point 1 (day 1/baseline), and Severity Score of other subjective symptoms would be 0, so that the mean value of the total score of Severity Score at observation point 1 (day 1/baseline) was assumed to be 0.5. In this study, it was also assumed that Severity Score did not worsen even at observation point 4 (day 14) and remained at 0.5 (amount of change of 0) in the *L. lactis* strain Plasma intake group. On the other hand, a recent meta-analytical results reported that the percentage of asymptomatic patients who become symptomatic is 48.9%[8], so it was assumed that the total score of Severity Score in the placebo intake group would worsen to about half that of day 0 in the above reference, and would be 5.5 (amount of change: 5). Since it is assumed that there are large individual differences in the degree of subjective symptoms when asymptomatic patients become symptomatic, when the standard deviation of the amount of change is supposed to be 7.0, which is slightly larger than that in the reference above, 42 cases per group would be required to detect a significant difference between groups at a significance level of 5% on both sides and detection power of 90%. Furthermore, assuming a dropout rate of about 15% of cases to be enrolled, the target number of cases to be enrolled was set at 50 cases per group and 100 cases per 2 groups.

2.12 Study period

Case enrollment period: from the date of jRCT publication to October 31, 2022
Observation period: 14 days

2.13 Data Quality Assurance

The monitoring work in this study was carried out in accordance with the monitoring procedure manual.

Regarding data quality control, the primary investigator checked the progress of the study as needed through a monitoring staff to confirm that the study was being conducted in accordance with the study protocol and the "Clinical Research Act (promulgated on April 14, 2017 and enforced on April 1, 2018)," and made sure that appropriate measures were taken to prevent deviations.

6. Course of treatment

The flow chart of the study is shown in Fig. 2. The numbers in parentheses indicate the number of relevant cases. In this study, 626 subjects were screened, and 100 patients were enrolled as planned in the protocol. The reasons for discontinuation and dropout cases in the *L. lactis* Plasma strain intake group were as follows.

- Discontinuation (1): Due to worsening of the primary disease or complications, it was determined that continuing to take the study foods was not advisable.
- Dropout (1): Due to discharge from a care facility, various subsequent tests cannot be carried out

Detailed information of the study subjects is also shown in Table 4.

[Table 4]

| Table 4: Study subjects breakdown | | |
|---|---|---|
| Subject | Number of cases | |
| | *L. lactis* strain Plasma intake group | Placebo intake group |
| Definitive enrollment, randomized cases | 51 | 49 |
| FAS | 50 | 46 |
| PPS | 45 | 46 |
| Safety analysis set | 51 | 48 |
| Cases having withdrawn the consent | 0 | 0 |
| Cases never subjected to study treatment after randomization | 0 | 1 |
| Cases of having at least one of the data of the primary endpoints* | 51 | 47 |
| Cases not matching the inclusion criteria | 1 | 3 |
| Cases conflicting with the exclusion criteria | 0 | 0 |
| * Amount of change in total score of Severity Score, Amount of change in total score of VAS | | |

6. Protocol Compliance

The protocol deviations in this study are as follows.

6.1 Major non-conformities

In this study, no cases that could be considered as "major non-conformities" were identified.

6.2 Other non-conformities

The following seven non-conformities were reported under "other non-conformities" (Table 5). Regarding these non-conformities, the Data Handling Committee decided in a blinded manner how to handle each piece of data.

[Table 5]

| Table 5: Non-conformity list | | |
|---|---|---|
| Category | Non-conformity contents | Incidence |
| 1. Deviation from eligibility criteria | Enrollment of a case deviating from inclusion criterion 1 "SARS-CoV-2 positive subject" was found by a test after the enrollment.<br>* Two cases of "a subject was institutionalized as a positive subject from the health department to the care facility and thus determined to match the eligibility and enrolled"<br>Two cases of "a subject was tested positive for SARS-Cov2 detection by prior antigen testing and thus determined to match the eligibility and enrolled" | 4 cases* |
| 2. Deviation from prohibited concomitant therapy | MIYA-BM (probiotics) was used for an adverse event (diarrhea). | 1 case |
| 3. Deviation from limited concomitant therapy | After the end of the prescription of acetaminophen, a study subject determined himself or herself to take loxoprofen (analgesia, antiinflammation, antipyretic) on hand. | 1 case |
| | A study subject used loxonin (analgesia, antiinflammation, antipyretic) for an adverse event "rib fracture due to persistent coughing." | 1 case |

(Reasons for being determined as a non-significant non-conformity**)

For 1, all cases were ineligible*** at enrollment because they were found to be negative by PCR testing after the start of the study, and thus were unavoidable deviations and treated as non-conformity.

For 2 and 3, these cases were deviations based on the study subject's own judgment and deviations to deal with adverse events, etc.

Based on the above, these deviations do not fall under the category of "Major non-conformities (see below)" as stipulated in the protocol, and were determined to be "Other non-conformities". The Data Handling Committee made a decision on the handling of the patient data in a blinded manner, and the following decision was made.

- For 1: The patient data were excluded from FAS and PPS.
- For 2: The patient data were excluded from PPS, but included in the other data sets.
- For 3: The patient data were included in all data sets.

** "Major non-conformities" as defined in this study (see "19.4 Management of non-conformities" in the study protocol)

"Major non-conformities" refers to those that affect the human rights and safety of the subjects of the clinical research and the progress and the reliability of the results of the study. However, it did not include cases in which the study protocol was not followed in order to avoid immediate danger to the subject of the clinical research or for other medically compelling reasons.

*** Ineligible at enrollment (See "19.4 Management of non-conformity" in the study protocol)

Information generated prior to enrollment according to the methods and criteria specified in the study protocol does not meet any of the inclusion criteria or meet any of the exclusion criteria. Cases, where information that occurred prior to enrollment is found to be incorrect after enrollment, are also included herein. This is treated as nonconforming.

7. Statistical Analysis

Techniques and the like of the statistical analysis in this study are shown below.

7.1 Analysis set

Full Analysis Set: FAS

Study subjects who were enrolled in this study and allocated to the study treatment were included in the full analysis set (FAS). However, data from study subjects with serious violations of the study protocol (failure to obtain consent, enrollment outside the contract period, etc.) were excluded.

Per Protocol Set: PPS

Study subjects were selected from FAS, excluding cases with the following serious violations of the provisions of the study protocol including the intake of the study foods and prohibited concomitant therapy, etc.

- Violation of inclusion criteria/exclusion criteria
- Violation of prohibited concomitant therapy
- When the intake rate of the study food exceeds 120% or is less than 75% of the total planned intake frequency

Safety analysis set

Those who were enrolled in the study, started to take the study foods, and took some or all of the study foods were determined to be study subjects.

7.2 Correspondence between statistical analysis items and analysis sets

The primary and secondary endpoints for efficacy were analyzed using FAS as the major analysis set of this study. If necessary, efficacy evaluation is analyzed using PPS as the sensitivity analysis of the FAS analysis results. Safety analysis was conducted using the safety analysis set as targets.

7.3 Data handling

- Data for which follow-up had been completed and fixed were included.
- Regarding duplicated registered data, unnecessary data were deleted, and then analysis was conducted.
- In principle, missing values are not supplemented.
- The handling of individual data was evaluated and decided in a blinded manner by the data handling committee that had been held prior to data fixation.
- For data less than or at the lower detection limit in the test values, half of each lower detection limit was substituted.
- The definitions of the amount of change and the rate of change were as follows.

Amount of change = post-intervention measurement - baseline measurement

$$\text{Rate of change} = (\text{amount of change/baseline measurement}) \times 100\ (\%)$$

- For the total score of Severity Score, 2 items of the 8 collected subjective symptoms, "no taste" and "no smell", are treated as 1 item (0 to 3 points) after integrating the data, obtaining the total points (0 to 21 points) of 7 items in total. For the integration of "no taste" and "no smell", the scores for the two items were summed, and all cases where the total value was 3 or higher points were treated as 3 points.
- Also for the total score of subjective symptom VAS, 2 items of the 8 collected subjective symptoms, "no taste" and "no smell", are treated as one item (0 to 10) after integrating the data, obtaining the total value (0 to 70) of 7 items in total. For the integration of "no taste" and "no smell", the values of the two items were summed, and all cases where the total value was 10 or higher were treated as 10.

7.4 Statistical analysis issues

- The significance level was set at 0.05 on both sides, and the reliability coefficient was set at 0.95 on both sides.
- No adjustment for multiplicity of tests was made.
- As summary statistics, the number of cases, mean, standard deviation, minimum value, first quartile, median, third quartile, and maximum value were calculated as continuous variables, and frequency and percentage (%) as categorical variables.

- In cases where data deviated significantly from a normal distribution, variable transformations were attempted as appropriate.

7.5 Detailed information of study subjects

The number of randomized cases, the number of cases in each of FAS, PPS, and safety analysis set, as well as the number of subjects who withdrew consent, subjects who never received study treatment after randomization, subjects having at least one of the data of primary endpoints, subjects not meeting the inclusion criteria, and subjects conflicting with the exclusion criteria were calculated for each group.

7.6 Analysis of the background of study subject

FAS was subjected to calculation such that summary statistics for the study subject background data (see below) were calculated for each group. For comparisons between groups, chi-square test and Fisher's exact test were carried out for categorical variables, and two-sample t-test was carried out for continuous variables. If a significant deviation from normal distribution was observed for continuous variables, the Wilcoxon rank sum test was carried out.

(Study subject background data)

Sex, age, height, body weight, BMI, with or without COVID-19 onset, with or without SARS-CoV-2 vaccination, and number of vaccinations

7.7 Analysis of primary endpoints

For changes in subjective symptoms, the primary endpoint, FAS and PPS were subjected to null hypothesis testing indicating that the amounts of change in both groups were equal, such that summary statistics were calculated for each group for the amount of change in the total score of Severity Score and the total score of VAS up to day 14, and then the null hypothesis testing was carried out using analysis of covariance. Covariates in the analysis of covariance were determined to be allocation adjustment factors (age of less than 50/50 or more years, taking or not taking anti-SARS-CoV-2 viral medication, and with or without SARS-CoV-2 vaccination) and corresponding baseline values. In addition, as a sensitivity analysis, longitudinal data analysis was carried out using the mixed effect model for repeated measures (MMRM) method. The factors used were allocation group, time, allocation group-to-time interaction, allocation adjustment factor, and corresponding baseline values as fixed effects, and the study subjects were used as variable effects. Unstructure was used for the correlation structure. However, Compound-Symmetry was used, when the calculation results did not converge.

7.8 Analysis of secondary endpoints

The analysis of secondary endpoints is described below. Since there was no difference in the levels of SARS-CoV-2 specific antibodies between the two groups, immune cell analysis was not carried out.

7.8.1 Endpoints for the amount of change and the rate of change

FAS and PPS were subjected to a two-sample t-test, such that summary statistics of the measurements and the amounts of change, and the rates of change at each observation point in the following endpoints were calculated for each group, and then the two-sample t-test was carried out for between-group comparison. Further, for the amount of change and the rate of change, a one-sample t-test was carried out for within-group comparison, and between-group comparison was also carried out by the MMRM method. In the MMRM analysis, the fixed effects were allocation group, time, allocation group-to-time interaction, allocation adjustment factor, and corresponding baseline value, and the variable effects were used as the study subjects. Unstructure was used as the correlation structure, and compound symmetry was used when the calculation results did not converge.

Target endpoints

- Amounts of change, rates of change of SARS-CoV-2 viral load

- Amounts of change and rates of change in the proportion and absolute number of pDCs and the same of activation markers (HLA-DR, CD86)
- Amounts of change and rates of change in SARS-CoV-2-specific antibody levels (IgM, IgG)
- Amounts of change and rates of change in blood cytokines (IL-6, MCP-1)
- Amounts of change and rates of change in interferon and interferon-inducible antiviral factors in peripheral blood mononuclear cells (PBMCs)

7.8.2 Proportion of outpatient visits and hospitalizations

FAS and PPS were subjected to between-group comparisons, such that the number and proportion of study subjects who had visited the outpatient department or had been hospitalized during the study period were compiled for each group, and then between-group comparisons were carried out using chi-square test and Fisher's exact test.

7.9 Analysis of safety endpoints (information of diseases, etc., and adverse events)

(Regarding the endpoint of the amount of change)

Safety analysis set was subjected to a two-sample t-test, such that summary statistics of the measurements and the amounts of change at each observation point in the following endpoints were calculated for each group, and then the two-sample t-test was carried out for between-group comparison. Further, for the amount of change, a one-sample t-test was carried out for within-group comparison, and between-group comparison was also carried out by the MMRM method. In the MMRM analysis, the fixed effects were allocation group, time, allocation group-to-time interaction, allocation adjustment factor, and corresponding baseline value, and the variable effects were used as the study subjects. Unstructure was used as the correlation structure, and compound symmetry was used when the calculation results did not converge.

(Regarding adverse events, diseases, etc.)

Safety analysis set was subjected to between-group comparison, such that the number and proportion of adverse events and serious adverse events occurring are compiled for each group, and then between-group comparison is carried out using Fisher's exact test.

7.10 General matters in data analysis

Statistical analysis software

The work to create charts or listings planned in this protocol is carried out using SAS version 9.4 on Windows 10, and the results are stored in PDF files, or Microsoft Word 2016 rich text files, or Excel 2016 worksheets. Validation of SAS programs created for individual analysis processes is not carried out.

General matters concerning the formats of charts or listings

The size of the charts is A4, with portrait as the standard for figures and summary tables, and landscape as the standard for listings and data lists, but these were determined according to the specifics of the individual charts.

Common rules for data processing

(1) Terms and conditions regarding the number of days and duration

- In calculating the number of days, add 1 to the number obtained by subtracting the start date from the end date.

   (For example) Number of dosing days: If dosing ended on the administration start date, the dosing period would be 1 day.
   Time to adverse event: If an adverse event occurred on the day following the administration start date, the time to the date where the first adverse event occurred would be 2 days.
   Survival period: If the patient died the day after the start of administration, survival period would be 2 days.

- When converting the number of days to that of years, months, or weeks, a year is converted to 365.25 days, a month is converted to 30.4375 days, a week is converted to 7 days, and so on.
- In the indication of "Time from the start of administration (day)" or "Time of onset (day)", the administration start date is counted as 1, the day before the start of administration is counted as -1, and so on, counting up or down by one day each in the same manner.

(2) Number of decimal places and rounding rules

- Percentages are expressed to one decimal place (e.g. 12.3%) and rounded to two decimal places.
- When calculating statistics such as mean, standard deviation, median, etc., rounding is not carried out during the calculation process, but rounding off is carried out in the final calculation result.

8. Study results_Evaluation of efficacy

The main results of this study are described below. For details of the analysis results, please refer to the Statistical Analysis Report (Version 1.0).

8.1 Study subject background information

- The backgrounds of study subjects of this study are shown in Table 6. There were no particularly significant differences among the groups in the background factors of the study subjects.

[Table 6]

Table 6-1: FAS continuous variable

| Variable | Statistic | *L. lactis* strain Plasma intake group | Placebo intake group | Difference between groups | Comparis on between groups P-value |
|---|---|---|---|---|---|
| Age | N | 50 | 46 | | |
| | Mean±SD | 38.1±13.5 | 38.7±12.5 | -0.6 | 0.83 |
| | 95%CI of Mean | 34.3, 42.0 | 35.0, 42.4 | -5.9, 4.7 | |
| | Median [Q1, Q3] | 37.5 [26.0, 49.0] | 40.5 [26.0, 50.0] | | |
| | Min, Max | 20.0, 64.0 | 20.0, 62.0 | | |
| Height (cm) | N | 50 | 46 | | |
| | Mean±SD | 163.5±8.6 | 166.1±7.6 | -2.7 | 0.11 |
| | 95%CI of Mean | 161.0, 165.9 | 163.9, 168.4 | -6.0, 0.6 | |
| | Median [Q1, Q3] | 163.8 [157.5, 170.0] | 167.5 [159.5, 172.5] | | |
| | Min, Max | 145.0, 182.0 | 151.0, 178.0 | | |
| Body weight (kg) | N | 50 | 46 | | |
| | Mean±SD | 61.7±10.5 | 64.0±10.0 | -2.2 | 0.29 |
| | 95%CI of Mean | 58.8, 64.7 | 61.0, 66.9 | -6.4, 2.0 | |
| | Median [Q1, Q3] | 60.6 [53.0, 68.3] | 63.6 [58.2, 71.0] | | |
| | Min, Max | 39.5, 89.0 | 39.8, 81.4 | | |
| BMI (kg/m²) | N | 50 | 46 | | |
| | Mean±SD | 23.1±3.3 | 23.1±3.0 | -0.1 | 0.92 |
| | 95%CI of Mean | 22.1, 24.0 | 22.2, 24.0 | -1.3, 1.2 | |
| | Median [Q1, Q3] | 23.0 [20.7, 25.1] | 23.2 [20.6, 25.5] | | |
| | Min, Max | 17.8, 29.9 | 15.9, 28.5 | | |

Table 6-2: FAS category variable

| Variable | Level | L. lactis strain Plasma intake group | | Placebo intake group | | Comparison between groups P-value | |
|---|---|---|---|---|---|---|---|
| | | N | Number of cases (%) | N | Number of cases (%) | χ-square | Fisher |
| Sex | Male | 50 | 27 (54.0) | 46 | 28 (60.9) | 0.50 | 0.54 |
| | Female | | 23 (46.0) | | 18 (39.1) | | |
| COVID-19 onset | Yes | 50 | 50 (100.0) | 46 | 46 (100.0) | - | - |
| SARS-CoV-2 vaccination | Yes | 50 | 42 (84.0) | 46 | 39 (84.8) | 0.92 | 1.00 |
| SARS-CoV-2 vaccination/n umber of vaccinations | 0 | 50 | 8 (16.0) | 46 | 7 (15.2) | NA | 1.00 |
| | 1 | | 0 (0.0) | | 0 (0.0) | | |
| | 2 | | 41 (82.0) | | 39 (84.8) | | |
| | 3 | | 1 (2.0) | | 0 (0.0) | | |
| A result that did not satisfy the requirements for carrying out χ-square test was denoted as NA. | | | | | | | |

8.3 Results of primary endpoints

The results of the primary endpoints are shown in Table 7. No significant differences were found between the groups.

[Table 7]

| Table 7-1 [FAS] Severity Score total score, VAS total score/Analysis of covariance | | | | | | | |
|---|---|---|---|---|---|---|---|
| Variable | | Observa tion point | L. lactis strain Plasma intake group | | Placebo intake group | | Difference in adjusted mean value (95%CI) | Comparis on between groups P-value |
| | | | N | Adjusted mean value(SE) | N | Adjusted mean value(SE) | | |
| Severity Score total score | Amount of change | day2 | 50 | -1.0 (0.4) | 45 | -1.3 (0.5) | 0.3 (-0.4, 1.0) | 0.41 |
| | | day3 | 50 | -1.8 (0.6) | 45 | -2.2 (0.6) | 0.4 (-0.5, 1.2) | 0.41 |
| | | day4 | 49 | -2.1 (0.6) | 45 | -2.2 (0.6) | 0.1 (-0.8, 1.0) | 0.84 |
| | | day5 | 49 | -2.0 (0.6) | 45 | -2.6 (0.6) | 0.6 (-0.4, 1.5) | 0.22 |
| | | day6 | 49 | -2.6 (0.6) | 45 | -2.9 (0.6) | 0.4 (-0.6, 1.3) | 0.45 |
| | | day7 | 49 | -2.9 (0.5) | 44 | -3.0 (0.5) | 0.1 (-0.7, 0.8) | 0.88 |
| | | day8 | 47 | -2.9 (0.5) | 45 | -3.1 (0.5) | 0.2 (-0.6, 0.9) | 0.64 |
| | | day9 | 47 | -3.3 (0.4) | 44 | -3.3 (0.4) | 0.0 (-0.6, 0.6) | 0.99 |
| | | day10 | 47 | -3.3 (0.4) | 44 | -3.1 (0.5) | -0.1 (-0.8, 0.5) | 0.67 |
| | | day11 | 47 | -3.5 (0.4) | 45 | -3.4 (0.4) | -0.2 (-0.8, 0.4) | 0.53 |
| | | day 12 | 47 | -3.3 (0.4) | 44 | -3.2 (0.4) | -0.1 (-0.7, 0.5) | 0.68 |
| | | day13 | 45 | -3.1 (0.4) | 45 | -3.1 (0.4) | -0.1 (-0.7, 0.5) | 0.83 |
| | | day14 | 45 | -3.5 (0.3) | 44 | -3.4 (0.4) | 0.0 (-0.6, 0.5) | 0.87 |

Table 7-1 [FAS] Severity Score total score, VAS total score/Analvsis of covariance

| Variable | | Observation point | L. lactis strain Plasma intake group | | Placebo intake group | | Difference in adjusted mean value (95%CI) | Comparison between groups P-value |
|---|---|---|---|---|---|---|---|---|
| | | | N | Adjusted mean value(SE) | N | Adjusted mean value(SE) | | |
| VAS total score | Amount of change | day2 | 50 | -2.3 (1.4) | 45 | -3.9 (1.4) | 1.6 (-0.5, 3.7) | 0.14 |
| | | day3 | 50 | -5.3 (1.6) | 45 | -6.8 (1.7) | 1.5 (-1.0, 4.0) | 0.24 |
| | | day4 | 49 | -6.1 (1.6) | 45 | -7.5 (1.6) | 1.3 (-1.1, 3.8) | 0.27 |
| | | day5 | 49 | -5.7 (1.8) | 45 | -7.9 (1.9) | 2.3 (-0.6, 5.1) | 0.11 |
| | | day6 | 49 | -6.7 (1.7) | 45 | -8.6 (1.8) | 1.9 (-0.8, 4.5) | 0.16 |
| | | day7 | 49 | -8.2 (1.3) | 44 | -9.3 (1.4) | 1.1 (-0.9, 3.2) | 0.27 0.11 |
| | | day8 | 47 | -7.5 (1.2) | 45 | -9.0 (1.2) | 1.5 (-0.3, 3.4) | |
| | | day9 | 47 | -9.0 (0.9) | 44 | -9.8 (0.9) | 0.7 (-0.7, 2.1) | 0.30 |
| | | day10 | 47 | -8.3 (0.9) | 44 | -9.1 (1.0) | 0.8 (-0.5, 2.1) | 0.23 |
| | | day11 | 47 | -8.6 (0.9) | 45 | -9.4 (0.9) | 0.8 (-0.6, 2.2) | 0.28 0.27 |
| | | day 12 | 47 | -8.3 (1.0) | 44 | -9.2 (1.0) | 0.9 (-0.7, 2.4) | |
| | | day13 | 45 | -8.0 (1.2) | 45 | -9.1 (1.2) | 1.1 (-0.8, 3.0) | 0.26 |
| | | day14 | 45 | -9.0 (0.9) | 44 | -9.8 (0.9) | 0.8 (-0.5, 2.2) | 0.23 |

Analysis of covariance was carried out using an allocation adjustment factor and the corresponding baseline value as covariates.

Table 7-2 [FAS] Severity Score total score, VAS total score/MMRM

| Variable | | Observation point | L. lactis strain Plasma intake group | | Placebo intake group | | Difference in adjusted mean value (95%CI) | Comparison between groups P-value |
|---|---|---|---|---|---|---|---|---|
| | | | N | Adjusted mean value(SE) | N | Adjusted mean value(SE) | | |
| Severity Score total score | Amount of Change | day2 | 50 | -0.6 (0.4) | 45 | -0.9 (0.4) | 0.2 (-0.7, 1.2) | 0.64 |
| | | day3 | | -1.2 (0.4) | | -1.5 (0.4) | 0.3 (-0.6, 1.3) | 0.52 |
| | | day4 | | -1.8 (0.4) | | -1.8 (0.4) | 0.0 (-0.9, 1.0) | 0.97 |
| | | day5 | | -1.8 (0.4) | | -2.3 (0.4) | 0.5 (-0.4, 1.5) | 0.29 |
| | | day6 | | -2.2 (0.4) | | -2.5 (0.4) | 0.3 (-0.6, 1.2) | 0.53 |
| | | day7 | | -2.6 (0.4) | | -2.7 (0.4) | 0.1 (-0.7, 0.8) | 0.88 |
| | | day8 | | -2.7 (0.4) | | -2.9 (0.4) | 0.2 (-0.6, 0.9) | 0.68 |
| | | day9 | | -3.1 (0.3) | | -3.1 (0.4) | 0.0 (-0.6, 0.7) | 0.97 |
| | | day10 | | -3.2 (0.3) | | -3.1 (0.4) | -0.1 (-0.8, 0.5) | 0.72 |
| | | day11 | | -3.3 (0.3) | | -3.1 (0.3) | -0.2 (-0.8, 0.4) | 0.57 |
| | | day12 | | -3.3 (0.3) | | -3.2 (0.3) | -0.1 (-0.7, 0.5) | 0.71 |
| | | day 13 | | -3.2 (0.3) | | -3.2 (0.3) | 0.0 (-0.7, 0.6) | 0.92 |
| | | day14 | | -3.4 (0.3) | | -3.3 (0.3) | -0.1 (-0.7, 0.5) | 0.71 |

(continued)

| Variable | | Observation point | L. lactis strain Plasma intake group | | Placebo intake group | | Difference in adjusted mean value (95%CI) | Comparison between groups P-value |
|---|---|---|---|---|---|---|---|---|
| | | | N | Adjusted mean value(SE) | N | Adjusted mean value(SE) | | |
| VAS total score | Amount of Change | day2 | 50 | -1.9 (1.3) | 45 | -4.1 (1.3) | 2.2 (-1.3, 5.7) | 0.21 |
| | | day3 | | -3.4 (1.2) | | -5.3 (1.3) | 1.9 (-1.4, 5.2) | 0.25 |
| | | day4 | | -5.0 (1.0) | | -6.5 (1.1) | 1.5 (-1.2, 4.2) | 0.28 |
| | | day5 | | -5.2 (1.1) | | -7.4 (1.1) | 2.2 (-0.7, 5.2) | 0.13 |
| | | day6 | | -5.9 (1.0) | | -7.7 (1.1) | 1.8 (-0.8, 4.5) | 0.18 |
| | | day7 | | -7.3 (0.8) | | -8.5 (0.9) | 1.2 (-0.9, 3.2) | 0.25 |
| | | day8 | | -7.4 (0.8) | | -8.9 (0.8) | 1.6 (-0.3, 3.4) | 0.10 |
| | | day9 | | -8.6 (0.6) | | -9.3 (0.7) | 0.7 (-0.6, 2.1) | 0.28 |
| | | day10 | | -8.7 (0.6) | | -9.4 (0.7) | 0.7 (-0.6, 2.0) | 0.30 |
| | | day11 | | -8.8 (0.7) | | -9.5 (0.7) | 0.7 (-0.7, 2.1) | 0.32 |
| | | day12 | | -8.9 (0.7) | | -9.6 (0.7) | 0.8 (-0.8, 2.3) | 0.33 |
| | | day 13 | | -8.7 (0.8) | | -9.7 (0.8) | 1.0 (-0.9, 2.9) | 0.28 |
| | | day14 | | -9.2 (0.6) | | -9.9 (0.7) | 0.6 (-0.7, 2.0) | 0.36 |
| Longitudinal data analysis was carried out by the MMRM method using: fixed effects including allocation group, time, allocation group-to-time interaction, allocation adjustment factor, and corresponding baseline value; variable effects including study subject; and Unstructured as correlation structure. | | | | | | | | |

Table 7-3 [FAS] Severity Score total score/summary statistic

| Variable | | Observation point | Statistic | *L. lactis* strain Plasma intake group | Placebo intake group |
|---|---|---|---|---|---|
| Severity Score total score | Amount of Change | day2 | N | 50 | 45 |
| | | | Mean±SD | -0.6±1.6 | -0.9±2.0 |
| | | | 95%CI of Mean | -1.0, -0.2 | -1.5, -0.3 |
| | | | Median [Q1, Q3] | 0.0 [-2.0, 0.0] | 0.0 [-2.0, 0.0] |
| | | | Min, Max | -5.0, 3.0 | -6.0, 3.0 |
| | | day3 | N | 50 | 45 |
| | | | Mean±SD | -1.2±2.3 | -1.6±2.6 |
| | | | 95%CI of Mean | -1.8, -0.5 | -2.3, -0.8 |
| | | | Median [Q1, Q3] | -1.0 [-2.0, 0.0] | -1.0 [-3.0, 0.0] |
| | | | Min, Max | -8.0,4.0 | -8.0, 3.0 |
| | | day4 | N | 49 | 45 |
| | | | Mean±SD | -1.8±2.5 | -1.9±2.7 |
| | | | 95%CI of Mean | -2.5, -1.1 | -2.7, -1.1 |
| | | | Median [Q1, Q3] | -1.0 [-3.0, 0.0] | -2.0 [-3.0, 0.0] |
| | | | Min, Max | -10.0,3.0 | -8.0,5.0 |
| | | day5 | N | 49 | 45 |
| | | | Mean±SD | -1.8±3.1 | -2.4±3.0 |
| | | | 95%CI of Mean | -2.7, -0.9 | -3.3, -1.5 |
| | | | Median [Q1, Q3] | -2.0 [-4.0, 0.0] | -2.0 [-5.0, 0.0] |
| | | | Min, Max | -10.0,8.0 | -8.0, 4.0 |
| | | day6 | N | 49 | 45 |
| | | | Mean±SD | -2.1±3.1 | -2.5±3.0 |
| | | | 95%CI of Mean | -3.0, -1.2 | -3.4, -1.6 |
| | | | Median [Q1, Q3] | -2.0 [-4.0, 0.0] | -2.0 [-5.0, 0.0] |
| | | | Min, Max | -11.0, 5.0 | -8.0,6.0 |
| | | day7 | N | 49 | 44 |
| | | | Mean±SD | -2.6±3.0 | -2.8±2.9 |
| | | | 95%CI of Mean | -3.5, -1.7 | -3.7, -1.9 |
| | | | Median [Q1, Q3] | -2.0 [-4.0, 0.0] | -2.0 [-5.5, 0.0] |
| | | | Min, Max | -11.0, 4.0 | -8.0, 3.0 |
| | | day8 | N | 47 | 45 |
| | | | Mean±SD | -2.7±3.2 | -3.0±2.9 |
| | | | 95%CI of Mean | -3.6, -1.8 | -3.8, -2.1 |
| | | | Median [Q1, Q3] | -2.0 [-4.0, 0.0] | -3.0 [-5.0, -1.0] |
| | | | Min, Max | -11.0, 4.0 | -8.0, 3.0 |
| | | day9 | N | 47 | 44 |
| | | | Mean±SD | -3.0±3.3 | -3.2±3.0 |
| | | | 95%CI of Mean | -4.0, -2.1 | -4.1, -2.3 |

(continued)

| Variable | | | Observation point | Statistic | *L. lactis* strain Plasma intake group | Placebo intake group |
|---|---|---|---|---|---|---|
| | | | | Median [Q1, Q3] | -2.0 [-5.0, -1.0] | -2.5 [-6.0, -1.0] |
| | | | | Min, Max | -12.0, 4.0 | -9.0, 2.0 |
| | | | day10 | N | 47 | 44 |
| | | | | Mean±SD | -3.2±3.4 | -3.1±2.9 |
| | | | | 95%CI of Mean | -4.2, -2.2 | -4.0, -2.2 |
| | | | | Median [Q1, Q3] | -2.0 [-5.0, -1.0] | -2.0 [-6.0, -1.0] |
| | | | | Min, Max | -12.0, 2.0 | -9.0,2.0 |
| | | | day11 | N | 47 | 45 |
| | | | | Mean±SD | -3.3±3.3 | -3.2±2.9 |
| | | | | 95%CI of Mean | -4.3, -2.3 | -4.1, -2.3 |
| | | | | Median [Q1, Q3] | -2.0 [-6.0, -1.0] | -2.0 [-6.0, -1.0] |
| | | | | Min, Max | -12.0, 2.0 | -9.0,2.0 |
| | | | day12 | N | 47 | 44 |
| | | | | Mean±SD | -3.3±3.2 | -3.2±2.8 |
| | | | | 95%CI of Mean | -4.2, -2.3 | -4.1, -2.4 |
| | | | | Median [Q1, Q3] | -2.0 [-6.0, -1.0] | -2.0 [-6.0, -1.0] |
| | | | | Min, Max | -11.0, 3.0 | -9.0, 1.0 |
| | | | day13 | N | 45 | 45 |
| | | | | Mean±SD | -3.1±3.4 | -3.2±2.8 |
| | | | | 95%CI of Mean | -4.1, -2.1 | -4.1, -2.4 |
| | | | | Median [Q1, Q3] | -2.0 [-5.0, -1.0] | -2.0 [-6.0, -1.0] |
| | | | | Min, Max | -12.0, 4.0 | -9.0, 1.0 |
| | | | day14 | N | 45 | 44 |
| | | | | Mean±SD | -3.4±3.3 | -3.4±2.9 |
| | | | | 95%CI of Mean | -4.4, -2.4 | -4.3, -2.5 |
| | | | | Median [Q1, Q3] | -2.0 [-6.0, -1.0] | -3.0 [-6.0, -1.0] |
| | | | | Min, Max | -12.0, 2.0 | -9.0, 1.0 |

Table 7-4 [FAS] VAS total score/summary statistic

| Variable | | | Observation point | Statistic | L. lactis strain Plasma intake group | Placebo intake group |
|---|---|---|---|---|---|---|
| VAS total score | | Amount of Change | day2 | N | 50 | 45 |
| | | | | Mean±SD | -2.3±6.9 | -3.5±5.1 |
| | | | | 95%CI of Mean | -4.2, -0.3 | -5.1, -2.0 |
| | | | | Median [Q1, Q3] | -0.5 [-2.0, 0.5] | -1.5 [-5.7, -0.5] |
| | | | | Min, Max | -36.2, 7.1 | -15.5, 5.8 |
| | | | day3 | N | 50 | 45 |
| | | | | Mean±SD | -3.8±8.8 | -4.8±7.0 |
| | | | | 95%CI of Mean | -6.3, -1.3 | -6.9, -2.7 |

(continued)

| Variable | | | Observation point | Statistic | L. lactis strain Plasma intake group | Placebo intake group |
|---|---|---|---|---|---|---|
| | | | | Median [Q1, Q3] | -2.2 [-4.9, 0.0] | -2.1 [-9.7, -0.3] |
| | | | | Min, Max | -41.2, 13.4 | -22.4, 6.8 |
| | | | day4 | N | 49 | 45 |
| | | | | Mean±SD | | -5.9±7.5 |
| | | | | 95%CI of Mean | -5.4±10.5 -8.5, -2.4 | -8.2, -3.7 |
| | | | | Median [Q1, Q3] | -2.7 [-8.1, -0.1] | -4.3 [-11.8, -1.0] |
| | | | | Min, Max | -48.3, 13.1 | -27.0, 10.6 |
| | | | day5 | N | 49 | 45 |
| | | | | Mean±SD | -5.7±12.5 | -6.9±9.0 |
| | | | | 95%CI of Mean | -9.3, -2.1 | -9.6, -4.2 |
| | | | | Median [Q1, Q3] | -4.4 [-10.7, -0.8] | -5.3 [-12.0, -1.2] |
| | | | | Min, Max | -50.4,33.0 | -35.4, 13.1 |
| | | | day6 | N | 49 | 45 |
| | | | | Mean±SD | -6.5±12.7 | -7.2±9.3 |
| | | | | 95%CI of Mean | -10.1, -2.8 | -10.0, -4.4 |
| | | | | Median [Q1, Q3] | -4.8 [-11.8, -0.1] | -5.3 [-13.6, -1.3] |
| | | | | Min, Max | -51.0,26.9 | -31.9, 14.6 |
| | | | day7 | N | 49 | 44 |
| | | | | Mean±SD | -7.8±11.7 | -8.0±9.6 |
| | | | | 95%CI of Mean | -11.2, -4.5 | -10.9, -5.1 |
| | | | | Median [Q1, Q3] | -5.8 [-13.1, -0.7] | -5.3 [-13.5, -1.9] |
| | | | | Min, Max | -51.2, 16.3 | -37.4, 9.2 |
| | | | day8 | N | 47 | 45 |
| | | | | Mean±SD | -7.5±12.4 | -8.4±9.5 |
| | | | | 95%CI of Mean | -11.1, -3.9 | -11.3, -5.5 |
| | | | | Median [Q1, Q3] | -5.1 [-14.2, 0.0] | -6.4 [-13.9, -1.9] |
| | | | | Min, Max | -51.1, 17.8 | -39.1, 8.7 |
| | | | day9 | N | 47 | 44 |
| | | | | Mean±SD | -8.8±11.8 | -8.9±9.8 |
| | | | | 95%CI of Mean | -12.3, -5.4 | -11.9, -5.9 |
| | | | | Median [Q1, Q3] | -6.2 [-14.2, -0.8] | -7.6 [-14.1, -1.9] |
| | | | | Min, Max | -51.4, 16.1 | -41.5, 8.9 |
| | | | day10 | N | 47 | 44 |
| | | | | Mean±SD | -8.9±11.9 | -8.5±9.2 |
| | | | | 95%CI of Mean | -12.4, -5.4 | -11.3, -5.7 |
| | | | | Median [Q1, Q3] | -7.0 [-14.2, -0.9] | -6.9 [-13.1, -2.0] |
| | | | | Min, Max | -51.4, 16.6 | -42.4, 7.2 |
| | | | day11 | N | 47 | 45 |
| | | | | Mean±SD | -9.0±12.1 | -9.0±9.0 |

(continued)

| Variable | | Observation point | Statistic | L. lactis strain Plasma intake group | Placebo intake group |
|---|---|---|---|---|---|
| | | | 95%CI of Mean | -12.5, -5.4 | -11.7, -6.3 |
| | | | Median [Q1, Q3] | -7.0 [-14.2, -0.9] | -6.1 [-14.2, -2.1] |
| | | | Min, Max | -51.4, 17.0 | -39.2, 0.0 |
| | | day 12 | N | 47 | 44 |
| | | | Mean±SD | -9.0±12.2 | -9.0±9.2 |
| | | | 95%CI of Mean | -12.6, -5.4 | -11.8, -6.2 |
| | | | Median [Q1, Q3] | -7.0 [-15.4, -1.0] | -7.0 [-14.5, -2.1] |
| | | | Min, Max | -50.7,24.8 | -39.4, 1.7 |
| | | day13 | N | 45 | 45 |
| | | | Mean±SD | | -9.2±9.4 |
| | | | 95%CI of Mean | -8.8±13.2 -12.7, -4.8 | -12.0, -6.3 |
| | | | Median [Q1, Q3] | -6.2 [-15.3, -1.1] | -6.0 [-14.2, -2.1] |
| | | | Min, Max | -51.4, 32.3 | -43.0, 0.4 |
| | | day14 | N | 45 | 44 |
| | | | Mean±SD | -9.5±12.2 | -9.3±9.7 |
| | | | 95%CI of Mean | -13.2, -5.8 | -12.2, -6.3 |
| | | | Median [Q1, Q3] | -7.0 [-14.8, -1.2] | -7.0 [-14.6, -2.0] |
| | | | Min, Max | -51.4, 16.6 | -43.2, 1.8 |

8.4 Results of secondary endpoints

8.4.1 Proportion and absolute number of pDCs and activation markers

The results of the proportion and absolute number of pDCs and activation markers are shown in Table 8. Both the proportion (rate of change) and absolute number (rate of change) of pDCs were significantly higher in the *L. lactis* strain Plasma intake group than those in the placebo intake group on day 8.

[Table 8]

| Table 8-1 [FAS] Proportion and absolute number of pDCs, and activation markers/MMRM | | | | | | | | |
|---|---|---|---|---|---|---|---|---|
| Variable | | Observation point | L. lactis strain Plasma intake group | | Placebo intake group | | Difference in adjusted mean value (95%CI) | Comparison between groups P-value |
| | | | N | Adjusted mean value(SE) | N | Adjusted mean value(SE) | | |
| Proportion of pDCs (%) | Amount of change | day4 | 49 | -0.00540 (0.00231) | 45 | -0.00653 (0.00234) | 0.00113 (-0.00271, 0.00497) | 0.56 |
| | | day8 | | -0.00430 (0.00245) | | -0.00760 (0.00249) | 0.00330 (-0.00120, 0.00780) | 0.15 |
| | Rate of change | day4 | 49 | -0.4 (17.8) | 45 | -17.9 (18.1) | 17.5 (-17.2, 52.1) | 0.32 |
| | | day8 | | -3.3 (15.8) | | -29.8 (16.0) | 26.5 (0.9, 52.2) | 0.042 |

(continued)

| Table 8-1 [FAS] Proportion and absolute number of pDCs, and activation markers/MMRM | | | | | | | | |
|---|---|---|---|---|---|---|---|---|
| Variable | | Obser vation point | L. lactis strain Plasma intake group | | Placebo intake group | | Difference in adjusted mean value (95%CI) | Compari son between groups P-value |
| | | | N | Adjusted mean value(SE) | N | Adjusted mean value(SE) | | |
| Absolute number of pDCs (/μL) | Amoun t of Change | day4 | 49 | -0.12 (0.11) | 45 | -0.23 (0.11) | 0.11 (-0.06, 0.28) | 0.22 |
| | | day8 | | 0.10 (0.14) | | -0.07 (0.14) | 0.17 (-0.12, 0.45) | 0.25 |
| | Rate of change | day4 | 49 | 21.1 (27.1) | 45 | -10.8 (27.4) | 32.0 (-13.6, 77.5) | 0.17 |
| | | day8 | | 48.0 (28.0) | | -1.8 (28.3) | 49.8 (0.1, 99.6) | 0.0498 |
| HLA-DR | Amoun t of change | day4 | 49 | 113.0 (152.1) | 45 | 191.8 (155.7) | -78.8 (-346.5, 188.8) | 0.56 |
| | | day8 | | -25.4 (150.9) | | 89.3 (154.7) | -114.7 (-378.1, 148.7) | 0.39 |
| | Rate of change | day4 | 49 | 15.8 (15.5) | 45 | 18.3 (15.9) | -2.5 (-29.3, 24.2) | 0.85 |
| | | day8 | | -0.6 (15.6) | | 14.8 (16.0) | -15.4 (-42.6, 11.8) | 0.26 |
| CD86 | Amoun t of change | day4 | 49 | 25.4 (79.8) | 45 | -43.2 (82.3) | 68.7 (-79.0, 216.3) | 0.36 |
| | | day8 | | -19.3 (77.5) | | 1.6 (80.0) | -20.9 (-158.8, 117.0) | 0.76 |
| | Rate of change | day4 | 49 | 84.4 (177.5) | 45 | -133.1 (183.3) | 217.4 (-138.9, 573.7) | 0.23 |
| | | day8 | | 99.4 (152.0) | | -63.3 (156.4) | 162.7 (-78.1, 403.5) | 0.18 |
| Longitudinal data analysis was carried out by the MMRM method using: fixed effects including allocation group, time, allocation group-to-time interaction, allocation adjustment factor, and corresponding baseline value; variable effects including study subject; and Unstructured as correlation structure. | | | | | | | | |

Table 8-2 [FAS] Proportion and absolute number of pDCs, and activation markers/summary statistic

| Variable | | Observ ation point | Statistic | L. lactis strain Plasma intake group | Placebo intake group | Differenc e between groups | Compariso n between groups P-value |
|---|---|---|---|---|---|---|---|
| Proportio | Amoun | day4 | N | 49 | 45 | | |

(continued)

| Variable | | Observation point | Statistic | L. lactis strain Plasma intake group | Placebo intake group | Difference between groups | Comparison between groups P-value |
|---|---|---|---|---|---|---|---|
| n of pDCs (%) | t of change | | Mean±SD | 0.00207±0.011 70 | 0.00715±0.016 91 | 0.00508 | 0.09 |
| | | | 95%CI of Mean | -0.00543, 0.00129 | -0.01223,-0.00207 | -0.00083, 0.01100 | |
| | | | Median [Q1, Q3] | -0.00100 [-0.00596, 0.00349] | -0.00600 [-0.01200, 0.00000] | | |
| | | | Min, Max | -0.03500, 0.03176 | -0.07100, 0.02400 | | |
| | | | One-sample t-test | 0.22 | 0.007 | | |
| | | day8 | N | 49 | 45 | | |
| | | | Mean±SD | 0.00097±0.012 02 | 0.00822±0.017 55 | 0.00725 | 0.021 |
| | | | 95%CI of Mean | -0.00442, 0.00248 | -0.01349, -0.00295 | 0.00113, 0.01337 | |
| | | | Median [Q1, Q3] | 0.00000 [-0.00458, 0.00427] | -0.00700 [-0.01183, -0.00100] | | |
| | | | Min, Max | -0.03900, 0.02800 | -0.08000, 0.02900 | | |
| | | | One-sample t-test | 0.57 | 0.003 | | |
| | Rate of change | day4 | N | 49 | 45 | | |
| | | | Mean±SD | 14.2±103.8 | -9.2±73.4 | 23.4 | 0.21 |
| | | | 95%CI of Mean | -15.6, 44.0 | -31.3, 12.8 | -13.7, 60.6 | |
| | | | Median [Q1, Q3] | -9.1 [-32.1, 32.0] | -36.4 [-59.3, 0.0] | | |
| | | | Min, Max | -85.3, 606.1 | -89.6, 203.7 | | |
| | | | One-sample t-test | 0.34 | 0.40 | | |
| | | day8 | N | 49 | 45 | | |
| | | | Mean±SD | 11.3±63.5 | -21.2±68.0 | 32.5 | 0.018 |
| | | | 95%CI of Mean | -6.9, 29.6 | -41.6, -0.8 | 5.6, 59.4 | |
| | | | Median [Q1, Q3] | 0.0 [-32.7, 46.5] | -42.9 [-63.8, - 11.3] | | |
| | | | Min, Max | -81.7, 180.2 | -100.0, 282.5 | | |
| | | | One-sample t-test | 0.22 | 0.042 | | |
| Absolute number | Amount of | day4 | N | 49 | 45 | | |
| | | | Mean±SD | 0.04±0.57 | -0.28±0.67 | 0.31 | 0.017 |

(continued)

| Variable | | Observation point | Statistic | L. lactis strain Plasma intake group | Placebo intake group | Difference between groups | Comparison between groups P-value |
|---|---|---|---|---|---|---|---|
| of pDCs(/μL) | change | | 95%CI of Mean | -0.13, 0.20 | -0.48, -0.07 | 0.06, 0.57 | |
| | | | Median [Q1, Q3] | 0.04 [-0.15, 0.26] | -0.14 [-0.49, 0.01] | | |
| | | | Min, Max | -1.83, 1.82 | -1.82, 1.08 | | |
| | | | One-sample t-test | 0.67 | 0.008 | | |
| | | day8 | N | 49 | 45 | | |
| | | | Mean±SD | 0.25±0.78 | -0.12±0.74 | 0.37 | 0.021 |
| | | | 95%CI of Mean | 0.03, 0.48 | -0.34, 0.10 | 0.06, 0.68 | |
| | | | Median [Q1, Q3] | 0.14 [-0.02, 0.42] | -0.13 [-0.52, 0.12] | | |
| | | | Min, Max | -1.52, 3.26 | -1.51, 2.05 | | |
| | | | One-sample t-test | 0.029 | 0.29 | | |
| | Rate of change | day4 | N | 49 | 45 | | |
| | | | Mean±SD | 49.1±130.2 | 3.5±102.9 | 45.6 | 0.06 |
| | | | 95%CI of Mean | 11.7, 86.5 | -27.4, 34.4 | -2.8, 93.9 | |
| | | | Median [Q1, Q3] | 13.5 [-21.1, 78.2] | -24.2 [-53.5, 1.3] | | |
| | | | Min, Max | -86.3, 686.7 | -93.5, 423.1 | | |
| | | | One-sample t-test | 0.011 | 0.82 | | |
| | | day8 | N | 49 | 45 | | |
| | | | Mean±SD | 75.9±138.5 | 12.5±111.4 | 63.4 | 0.017 |
| | | | 95%CI of Mean | 36.2, 115.7 | -20.9, 46.0 | 11.6, 115.2 | |
| | | | Median [Q1, Q3] | 38.3 [-11.8, 100.0] | -22.7 [-45.6, 22.6] | | |
| | | | Min, Max | -71.2, 596.0 | -100.0, 503.1 | | |
| | | | One-sample t-test | <0.001 | 0.45 | | |

(continued)

| Variable | | Observation point | Statistic | L. lactis strain Plasma intake group | Placebo intake group | Difference between groups | Comparison between groups P-value |
|---|---|---|---|---|---|---|---|
| HLA-DR | Amount of change | day4 | N | 49 | 45 | | |
| | | | Mean±SD | 216.0±707.5 | 352.8±667.9 | -136.8 | 0.34 |
| | | | 95%CI of Mean | 12.8,419.2 | 152.1, 553.4 | -419.2, 145.7 | |
| | | | Median [Q1, Q3] | 137.0 [-160.0, 638.0] | 179.0 [-123.0, 795.0] | | |
| | | | Min, Max | -1538.0, 1950.0 | -1004.0, 1943.0 | | |
| | | | One-sample t-test | 0.038 | <0.001 | | |
| | | day8 | N | 49 | 44 | | |
| | | | Mean±SD | 77.6±654.7 | 245.6±697.9 | -168.0 | 0.23 |
| | | | 95%CI of Mean | -110.4, 265.7 | 33.5, 457.8 | -446.7, 110.7 | |
| | | | Median [Q1, Q3] | -81.0 [-243.0, 216.0] | 102.0 [-155.5, 829.0] | | |
| | | | Min, Max | -1364.0, 1663.0 | -1325.0, 1705.0 | | |
| | | | One-sample t-test | 0.41 | 0.024 | | |
| | Rate of change | day4 | N | 49 | 45 | | |
| | | | Mean±SD | 30.5±70.1 | 38.3±67.8 | -7.8 | 0.59 |
| | | | 95%CI of Mean | 10.4, 50.7 | 17.9, 58.6 | -36.1, 20.5 | |
| | | | Median [Q1, Q3] | 12.1 [-17.0, 77.0] | 19.4 [-17.9, 97.9] | | |
| | | | Min, Max | -66.5, 296.4 | -65.4, 239.3 | | |
| | | | One-sample t-test | 0.004 | <0.001 | | |
| | | day8 | N | 49 | 44 | | |
| | | | Mean±SD | 14.2±62.3 | 34.5±74.1 | -20.3 | 0.16 |
| | | | 95%CI of Mean | -3.7,32.1 | 11.9, 57.0 | -48.4, 7.8 | |
| | | | Median [Q1, Q3] | -7.4 [-24.0, 26.2] | 13.5 [-17.9, 83.2] | | |
| | | | Min, Max | -66.3, 164.8 | -70.3, 213.0 | | |
| | | | One-sample t-test | 0.12 | 0.004 | | |

(continued)

| Variable | | Observation point | Statistic | L. lactis strain Plasma intake group | Placebo intake group | Difference between groups | Comparison between groups P-value |
|---|---|---|---|---|---|---|---|
| CD86 | Amount of Change | day4 | N | 49 | 45 | | |
| | | | Mean±SD | 16.8±497.6 | 23.3±564.7 | -6.5 | 0.95 |
| | | | 95%CI of Mean | -126.2, 159.7 | -146.4, 193.0 | -224.2, 211.1 | |
| | | | Median [Q1, Q3] | 59.0 [-284.0, 297.0] | 138.0 [-320.0, 391.1] | | |
| | | | Min, Max | -1262.0, 1138.0 | -1290.0, 1096.5 | | |
| | | | One-sample t-test | 0.81 | 0.78 | | |
| | | day8 | N | 49 | 44 | | |
| | | | Mean±SD | -28.0±488.6 | 58.4±492.8 | -86.4 | 0.40 |
| | | | 95%CI of Mean | -168.3, 112.3 | -91.4, 208.2 | -288.8, 116.0 | |
| | | | Median [Q1, Q3] | -29.0 [-285.0, 292.0] | 44.0 [-398.5, 430.0] | | |
| | | | Min, Max | -1401.0, 1056.0 | -863.0, 938.0 | | |
| | | | One-sample t-test | 0.69 | 0.44 | | |
| | Rate of change | day4 | N | 49 | 45 | | |
| | | | Mean±SD | 92.2±382.3 | -108.3±1158.9 | 200.5 | 0.26 |
| | | | 95%CI of Mean | -17.6, 202.0 | -456.4, 239.9 | -147.2, 548.1 | |
| | | | Median [Q1, Q3] | 6.7 [-29.7, 70.9] | 16.7 [-42.4, 83.7] | | |
| | | | Min, Max | -66.9, 2605.1 | -7562.1, 1353.3 | | |
| | | | One-sample t-test | 0.10 | 0.53 | | |
| | | day8 | N | 49 | 44 | | |
| | | | Mean±SD | 107.2±542.3 | -44.5±608.4 | 151.7 | 0.21 |
| | | | 95%CI of Mean | -48.5, 263.0 | -229.5, 140.4 | -85.3, 388.7 | |
| | | | Median [Q1, Q3] | -3.9 [-30.5, 47.1] | -2.3 [-41.9, 69.8] | | |
| | | | Min, Max | -82.8, 3743.3 | -3644.8, 1136.6 | | |
| | | | One-sample t-test | 0.17 | 0.63 | | |

8.4.2 SARS-CoV-2 viral load

The results of SARS-CoV-2 viral load are shown in Table 9. No significant differences were found between the groups.

However, the rate of change of the log-transformed SARS-CoV-2 (amount) was significantly decreased on day 4 in the *L. lactis* strain Plasma intake group (within group).

[Table 9]

Table 9-1 [FAS] SARS-CoV-2 viral load/MMRM

| Variable | | Observation point | *L. lactis* strain Plasma intake group | | Placebo intake group | | Difference in adjusted mean value (95%CI) | Comparison between groups P-value |
|---|---|---|---|---|---|---|---|---|
| | | | N | Adjusted mean value(SE) | N | Adjusted mean value(SE) | | |
| Log-transformed SARS-CoV-2 level(ln(Copies/5μL)) | Amount of change | day4 | 49 | -3.03 (0.75) | 46 | -2.55 (0.77) | -0.48 (-1.90, 0.93) | 0.50 |
| | | day8 | | -8.52 (0.68) | | -8.94 (0.70) | 0.41 (-0.70, 1.52) | 0.46 |
| | Rate of change | day4 | 49 | -22.5 (10.3) | 45 | -6.5 (10.6) | -16.0 (-39.5, 7.4) | 0.18 |
| | | day8 | | -67.3 (7.3) | | -67.1 (7.6) | -0.2 (-11.3, 11.0) | 0.97 |

Longitudinal data analysis was carried out by the MMRM method using: fixed effects including allocation group, time, allocation group-to-time interaction, allocation adjustment factor, and corresponding baseline value; variable effects including study subject; and Unstructured as correlation structure.

Table 9-2 [FAS] SARS-CoV-2 viral load/summary statistic and t-test

| Variable | | Observation point | Statistic | *L. lactis* strain Plasma intake group | Placebo intake group | Difference between groups | Comparison between groups P-value |
|---|---|---|---|---|---|---|---|
| SARS-CoV-2 (Copies/5μL) | Amount of change | day4 | N | 49 | 46 | | |
| | | | Mean±SD | -2199102.59 ± 5219847.83 | -1501737.62 ± 5832018.62 | | |
| | | | 95%CI of Mean | -3698417.95, -699787.24 | -3233633.37, 230158.13 | | |
| | | | Median [Q1, Q3] | -511140.00 [-2339020.30, -30079.30] | -377054.60 [-2710684.00, -5280.00] | | |
| | | | Min, Max | -26848582.40, 7672887.00 | -25583545.00, 20722960.00 | | |
| | | day8 | N | 49 | 46 | | |
| | | | Mean±SD | -2578395.74 ±4865157.89 | -2318837.30 ± 4537243.70 | | |
| | | | 95%CI of Mean | -3975832.25, -1180959.23 | -3666232.36, -971442.24 | | |
| | | | Median [Q1, Q3] | -532710.00 [-2623982.50, -105610.00] | -515321.00 [-2743258.51, -44403.40] | | |
| | | | Min, Max | -26848055.70, 7557.00 | -25633777.60, 2231.60 | | |

(continued)

| Variable | | Observation point | Statistic | *L. lactis* strain Plasma intake group | Placebo intake group | Difference between n groups | Comparison between groups P-value |
|---|---|---|---|---|---|---|---|
| | Rate of change | day4 | N | 49 | 45 | | |
| | | | Mean±SD | 921.6±6171.8 | 44307.8±25927 5.6 | | |
| | | | 95%CI of Mean | -851.1, 2694.4 | -33587.2, 122202.8 | | |
| | | | Median [Q1, Q3] | -96.5 [-99.8, -84.0] | -98.7 [-99.6, - 21.3] | | |
| | | | Min, Max | -100.0, 43074.6 | -100.0, 1730657.6 | | |
| | | day8 | N | 49 | 45 | | |
| | | | Mean±SD | -96.2±23.4 | -82.1±69.1 | | |
| | | | 95%CI of Mean | -102.9, -89.4 | -102.9, -61.4 | | |
| | | | Median [Q1, Q3] | -100.0 [-100.0, - 100.0] | -100.0 [-100.0, -100.0] | | |
| | | | Min, Max | -100.0, 64.0 | -100.0, 253.0 | | |
| Log-transformed SARS-CoV-2 level (ln (copies/5μL )) | Amount of change | day4 | N | 49 | 46 | | |
| | | | Mean±SD | -3.85±3.79 | -2.87±4.71 | -0.98 | 0.26 |
| | | | 95%CI of Mean | -4.94, -2.76 | -4.27, -1.47 | -2.72, 0.75 | |
| | | | Median [Q1, Q3] | -3.35 [-6.06, - 1.83] | -4.29 [-5.61, - 0.22] | | |
| | | | Min, Max | -13.22, 6.07 | -10.74, 9.62 | | |
| | | | One-sample t-test | <0.001 | <0.001 | | |
| | | day8 | N | 49 | 46 | | |
| | | | Mean±SD | -9.34±3.30 | -9.26±3.99 | -0.09 | 0.91 |
| | | | 95%CI of Mean | -10.29, -8.39 | -10.44, -8.07 | -1.57, 1.40 | |
| | | | Median [Q1, Q3] | -9.76 [-11.67, - 7.43] | -10.25 [-11.73, - 8.02] | | |
| | | | Min, Max | -15.05, 0.49 | -14.70, 1.16 | | |
| | | | One-sample t-test | <0.001 | <0.001 | | |
| | Rate of change | day4 | N | 49 | 45 | | |
| | | | Mean±SD | -27.9±29.7 | -10.7±84.2 | -17.2 | 0.18 |

(continued)

| Variable | | | Observation point | Statistic | *L. lactis* strain Plasma intake group | Placebo intake group | Difference between groups | Comparison between groups P-value |
|---|---|---|---|---|---|---|---|---|
| | | | | 95%CI of Mean | -36.4, -19.3 | -36.0, 14.6 | -42.7, 8.2 | |
| | | | | Median [Q1, Q3] | -25.1 [-43.5, -14.8] | -32.5 [-43.9, - 1.7] | | |
| | | | | Min, Max | -87.9, 62.0 | -88.2, 474.2 | | |
| | | | | One-sample t-test | <0.001 | 0.40 | | |
| | | | day8 | N | 49 | 45 | | |
| | | | | Mean±SD | -72.7±24.7 | -71.3±32.4 | -1.4 | 0.82 |
| | | | | 95%CI of Mean | -79.8, -65.6 | -81.0, -61.6 | -13.1, 10.3 | |
| | | | | Median [Q1, Q3] | -78.1 [-92.0, -59.9] | -78.4 [-89.8, - 63.3] | | |
| | | | | Min, Max | -100.0, 5.3 | -100.0, 57.3 | | |
| | | | | One-sample t-test | <0.001 | <0.001 | | |

9. Evaluation of efficacy

No significant difference was observed between groups in Severity Score, the primary endpoint in this analysis. However, on and after day 10, it was observed that the adjusted amount of change in the total score of Severity Score tended to be numerically smaller in the *L. lactis* strain Plasma intake group than that in the placebo intake group.

Moreover, pDCs are immune cells that play an important command post role when bacteria or viruses enter the body, and there is a study report that pDCs in the blood of COVD-19 patients are greatly reduced and do not easily recover thereafter[9], however, both the rate of change of the proportion of pDCs and the same of the absolute number of pDCs on day 8 were significantly higher in the *L. lactis* strain Plasma intake group than those in the placebo intake group.

For the amount of SARS-CoV-2, no significant difference was observed between groups in the amount of change, while the rate of change in SARS-CoV-2 (amount) was significantly decreased on day 4 in the *L. lactis* strain Plasma intake group (within groups).

10. Study results_Safety evaluation

The results of this study are as described below. For details of the analysis results, please refer to the Statistical Analysis Report (Version 1.0).

10.1 Vital signs

The results of vital signs are shown in Table 10. Compared to the *L. lactis* strain Plasma intake group, the placebo intake group tended to have a decreased pulse rate on day 2 and a significantly higher $SpO_2$ (%) on day 8, but overall there were no safety concerns between the groups.

[Table 10]

| Table 10 [Safety analysis set] vital sien/MMRM | | | | | | | |
|---|---|---|---|---|---|---|---|
| Variable | | Obser vation point | *L. lactis* strain Plasma intake group | | Placebo intake group | | Difference in adjusted mean value (95%CI) | Compari son between groups P-value |
| | | | N | Adjusted mean value(SE) | N | Adjusted mean value(SE) | | |
| Body tem-perature (de-gree) | Amount of change | day2 | 51 | -0.2 (0.1) | 48 | -0.2 (0.1) | 0.0 (-0.1, 0.1) | 0.92 |
| | | day3 | | -0.3 (0.1) | | -0.2 (0.1) | 0.0 (-0.2, 0.1) | 0.68 |
| | | day4 | | -0.3 (0.1) | | -0.3 (0.1) | 0.0 (-0.1, 0.1) | 0.81 |
| | | day5 | | -0.3 (0.1) | | -0.3 (0.1) | 0.0 (-0.1, 0.1) | 0.52 |
| | | day6 | | -0.3 (0.1) | | -0.2 (0.1) | -0.1 (-0.2, 0.1) | 0.27 |
| | | day7 | | -0.4 (0.1) | | -0.3 (0.1) | -0.1 (-0.2, 0.1) | 0.34 |
| | | day8 | | -0.3 (0.1) | | -0.3 (0.1) | 0.0 (-0.1, 0.1) | 0.74 |
| Pulse (beats/-min) | Amount of change | day2 | 51 | -1.0 (2.1) | 48 | -5.0 (2.1) | 4.0(0.5, 7.6) | 0.027 |
| | | day3 | | -3.9 (2.1) | | -5.6 (2.1) | 1.7 (-1.9, 5.3) | 0.35 |
| | | day4 | | -7.0 (2.1) | | -5.5 (2.2) | -1.4 (-5.2, 2.3) | 0.45 |
| | | day5 | | -4.5 (2.1) | | -6.0 (2.1) | 1.6 (-2.0, 5.1) | 0.38 |
| | | day6 | | -5.5 (2.1) | | -5.9 (2.1) | 0.5 (-3.1, 4.1) | 0.79 |
| | | day7 | | -3.7 (2.2) | | -5.3 (2.2) | 1.6 (-2.4, 5.6) | 0.43 |
| | | day8 | | -4.3 (2.2) | | -6.3 (2.3) | 2.0 (-2.1, 6.1) | 0.34 |

(continued)

| Table 10 [Safety analysis set] vital sien/MMRM | | | | | | | | |
|---|---|---|---|---|---|---|---|---|
| Variable | | Observation point | L. lactis strain Plasma intake group | | Placebo intake group | | Difference in adjusted mean value (95%CI) | Comparison between groups P-value |
| | | | N | Adjusted mean value(SE) | N | Adjusted mean value(SE) | | |
| SpO$_2$ (%) | Amount of Change | day2 | 51 | 0.0 (0.2) | 48 | 0.3 (0.2) | -0.2 (-0.6, 0.1) | 0.13 |
| | | day3 | | 0.0 (0.2) | | 0.1 (0.2) | -0.1 (-0.5, 0.2) | 0.50 |
| | | day4 | | 0.2 (0.2) | | 0.2 (0.2) | -0.1 (-0.4, 0.2) | 0.65 |
| | | day5 | | 0.2 (0.2) | | 0.2 (0.2) | 0.0 (-0.3, 0.3) | 0.94 |
| | | day6 | | 0.2 (0.2) | | 0.3 (0.2) | -0.1 (-0.4, 0.3) | 0.74 |
| | | day7 | | 0.2 (0.2) | | 0.4 (0.2) | -0.2 (-0.5, 0.1) | 0.26 |
| | | day8 | | 0.1 (0.2) | | 0.5 (0.2) | -0.4 (-0.8, -0.1) | 0.005 |
| Respiratory rate (breaths/min) | Amount of change | day4 | 50 | 0.0 (0.2) | 48 | -0.2 (0.2) | 0.2 (-0.1, 0.6) | 0.20 |
| | | day 8 | | 0.0 (0.3) | | -0.3 (0.3) | 0.3 (-0.2, 0.9) | 0.26 |
| Longitudinal data analysis was carried out by the MMRM method using: fixed effects including allocation group, time, allocation group-to-time interaction, allocation adjustment factor, and corresponding baseline value; variable effects including study subjects; and Unstructured as correlation structure. | | | | | | | | |

10.2 Blood test

Blood test results are shown in Table 11. No particular concerns were found in blood tests.

[Table 11]

| Table 11 [Safety analysis set] blood test/MMRM | | | | | | | | |
|---|---|---|---|---|---|---|---|---|
| Variable | | Observation point | L. lactis strain Plasma intake group | | Placebo intake group | | Difference in adjusted mean value (95%CI) | Comparison between groups P-value |
| | | | N | Adjusted mean value(SE) | N | Adjusted mean value(SE) | | |
| Neutrophil (%) | Amount of change | day4 | 46 | 3.93 (2.28) | 47 | 5.57 (2.24) | -1.64 (-5.15, 1.86) | 0.35 |
| | | day8 | | 9.33 (2.22) | | 9.78 (2.17) | -0.45 (-3.62, 2.73) | 0.78 |
| Lymphocyte (%) | Amount of change | day4 | 46 | -1.20 (2.07) | 47 | -2.72 (2.03) | 1.52 (-1.70, 4.73) | 0.35 |
| | | day8 | | -5.51 (2.01) | | -5.52 (1.97) | 0.02 (-2.86, 2.89) | 0.99 |

(continued)

| Table 11 [Safety analysis set] blood test/MMRM | | | | | | | | |
|---|---|---|---|---|---|---|---|---|
| Variable | | Observation point | *L. lactis* strain Plasma intake group | | Placebo intake group | | Difference in adjusted mean value (95%CI) | Comparison between groups P-value |
| | | | N | Adjusted mean value(SE) | N | Adjusted mean value(SE) | | |
| Log-transformed Eosinophil (ln(%)) | Amount of change | day4 | 46 | 0.17 (0.10) | 47 | 0.21 (0.10) | -0.04 (-0.19, 0.10) | 0.57 |
| | | day8 | | 0.20 (0.10) | | 0.20 (0.10) | 0.00 (-0.15, 0.14) | 0.96 |
| Monocyte (%) | Amount of change | day4 | 46 | -2.58 (0.40) | 47 | -3.09 (0.40) | 0.51 (-0.22, 1.23) | 0.17 |
| | | day8 | | -3.81 (0.36) | | -4.25 (0.35) | 0.44 (-0.07, 0.95) | 0.09 |
| Basophil (%) | Amount of change | day4 | 46 | -0.07 (0.06) | 47 | -0.11 (0.06) | 0.04 (-0.05, 0.14) | 0.35 |
| | | day8 | | -0.03 (0.05) | | -0.08 (0.05) | 0.05 (-0.03, 0.13) | 0.20 |
| Blood platelet ($\times 10^4/\mu$L) | Amount of change | day4 | 50 | 1.8 (1.0) | 48 | 1.5 (1.0) | 0.3 (-1.3, 1.8) | 0.73 |
| | | day8 | | 5.9 (1.1) | | 5.6 (1.1) | 0.2 (-1.6, 2.0) | 0.82 |
| Log-transformed AST (ln(U/L)) | Amount of change | day4 | 50 | -0.15 (0.05) | 48 | -0.16 (0.05) | 0.01 (-0.06, 0.08) | 0.80 |
| | | day8 | | -0.11 (0.06) | | -0.11 (0.06) | 0.00 (-0.11, 0.10) | 0.96 |
| Log-transformed ALT (ln(U/L)) | Amount of change | day4 | 50 | -0.06 (0.06) | 48 | -0.08 (0.06) | 0.02 (-0.07, 0.12) | 0.62 |
| | | day8 | | 0.05 (0.07) | | 0.00 (0.08) | 0.05 (-0.09, 0.20) | 0.48 |
| Log-transformed γ-GTP (ln(U/L)) | Amount of change | day4 | 50 | 0.03 (0.04) | 48 | 0.01 (0.04) | 0.02 (-0.04, 0.08) | 0.47 |
| | | day8 | | 0.02 (0.05) | | -0.01 (0.05) | 0.03 (-0.08, 0.15) | 0.55 |
| BUN (mg/dL) | Amount of change | day4 | 50 | -1.4 (0.6) | 48 | -0.7 (0.6) | -0.7 (-1.6, 0.2) | 0.11 |
| | | day8 | | -1.0 (0.6) | | -0.7 (0.6) | -0.3 (-1.3, 0.7) | 0.55 |
| Cre (mg/dL) | Amount of change | day4 | 50 | -0.05 (0.02) | 48 | -0.05 (0.02) | 0.00 (-0.03, 0.03) | 0.83 |
| | | day8 | | -0.04 (0.02) | | -0.05 (0.02) | 0.01 (-0.02, 0.04) | 0.33 |
| LDH (U/L) | Amount of change | day4 | 50 | -17.5 (6.0) | 48 | -14.4 (6.1) | -3.1 (-12.3, 6.0) | 0.50 |
| | | day8 | | -13.9 (6.3) | | -10.8 (6.4) | -3.1 (-13.7, 7.5) | 0.56 |

(continued)

| Table 11 [Safety analysis set] blood test/MMRM | | | | | | | | |
|---|---|---|---|---|---|---|---|---|
| Variable | | Observa tion point | *L. lactis* strain Plasma intake group | | Placebo intake group | | Difference in adjusted mean value (95%CI) | Compar ison between groups P-value |
| | | | N | Adjusted mean value(SE) | N | Adjusted mean value(SE) | | |
| Log-trans-formed CRP | Amount of change | day4 | 50 | -1.16 (0.20) | 48 | -1.11 (0.20) | -0.05 (-0.35, 0.25) | 0.75 |
| | | day8 | | -1.96 (0.22) | | -2.09 | 0.12 (-0.26, | 0.52 |
| (In(mg/dL)) | | | | | | (0.22) | 0.51) | |
| Log-trans-formed ferri-tin (In(ng/mL)) | Amount of change | day4 | 50 | -0.06 (0.07) | 48 | -0.05 (0.07) | -0.01 (-0.11, 0.09) | 0.86 |
| | | day8 | | 0.02 (0.08) | | 0.04 (0.08) | -0.02 (-0.16, 0.12) | 0.79 |
| HbAlc (%) | Amount of change | day4 | 50 | 0.1 (0.0) | 48 | 0.0 (0.0) | 0.0 (0.0, 0.1) | 0.43 |
| | | day8 | | 0.1 (0.0) | | 0.0 (0.0) | 0.1 (0.0, 0.1) | 0.16 |
| HbA1c (mmol/mol) | Amount of change | day4 | 50 | 0.6 (0.4) | 48 | 0.3 (0.5) | 0.3 (-0.4, 1.0) | 0.43 |
| | | day8 | | 0.9 (0.5) | | 0.3 (0.5) | 0.6 (-0.2, 1.4) | 0.16 |
| Longitudinal data analysis was carried out by the MMRM method using: fixed effects including allocation group, time, allocation group-to-time interaction, allocation adjustment factor, and corresponding baseline value; variable effects including study subjects; and Unstructured as correlation structure. | | | | | | | | |

10.3 Adverse event

All adverse events were mild and no adverse events of concern were observed.

11. Safety evaluation

In this study, no safety concerns associated with the intake of the *L. lactis* strain Plasma were observed.

12. Overview

This study is an extremely rare study in which the foods' effect of preventing the onset of and easing the symptoms of COVID-19 could be examined using the same strain of COVID-19 (Omicron strain). However, no significant difference was observed between groups in the primary endpoint, Severity Score, and secondary endpoints compared to placebo, but both the rate of change of the proportion and the same of the absolute number of pDCs on day 8 were significantly higher in the L. *lactis* strain Plasma intake group than those in the placebo intake group (Table 8-1 and Fig. 4). Also for the amount of SARS-CoV-2, no significant difference was observed between the groups in the amount of change, while the rate of change of SARS-CoV-2 (amount) was significantly decreased (within groups) on day 4 in the *L. lactis* strain Plasma intake group (Table 9-1 and Fig. 5). Additionally, no safety concerns were identified with the intake of the *L. lactis* strain Plasma in this study.

13. Analysis of individual subjective symptom scores

The above test data were analyzed for the secondary endpoints, individual subjective symptom scores (no taste score, no smell score, and sum of no taste score and no smell score).

- Analysis items

Secondary endpoints_Individual subjective symptom scores (no taste score, no smell score, sum of no taste score and no smell score)

- Analysis method

Following the instructions in "7. Statistical Analysis", statistical analysis was carried out using chi-square test on the number of subjects with a score of 0 and those with scores ranging from 1 to 3 for each day.

- Analysis results

Analysis results are shown in Table 12. In Table 12, "No taste + no smell score" refers to "the sum of no taste score and no smell score".

[Table 12]

Table 12: Analysis results    Secondary endpoints    Individual Subjective symptoms scores (no taste score, no smell score, no taste + no smell score)

**(1) No taste score**

| No taste score | | day1 (0) | day1 (1~3) | day2 (0) | day2 (1~3) | day3 (0) | day3 (1~3) | day4 (0) | day4 (1~3) | day5 (0) | day5 (1~3) | day6 (0) | day6 (1~3) | day7 (0) | day7 (1~3) |
|---|---|---|---|---|---|---|---|---|---|---|---|---|---|---|---|
| Group 1 | LC-Plasma | 44 | 6 | 44 | 6 | 43 | 7 | 41 | 8 | 40 | 9 | 40 | 9 | 43 | 6 |
| | Placebo | 43 | 3 | 42 | 4 | 41 | 5 | 40 | 6 | 40 | 6 | 40 | 6 | 41 | 5 |

| No taste score | | day8 (0) | day8 (1~3) | day9 (0) | day9 (1~3) | day10 (0) | day10 (1~3) | day11 (0) | day11 (1~3) | day12 (0) | day12 (1~3) | day13 (0) | day13 (1~3) | day14 (0) | day14 (1~3) |
|---|---|---|---|---|---|---|---|---|---|---|---|---|---|---|---|
| Group 2 | LC-Plasma | 41 | 6 | 44 | 3 | 44 | 3 | 44 | 3 | 44 | 3 | 44 | 1 | 44 | 1 |
| | Placebo | 40 | 6 | 39 | 6 | 40 | 5 | 41 | 5 | 40 | 5 | 41 | 5 | 41 | 4 |

day13 p=0.09

**(2) No smell score**

| No smell score | | day1 (0) | day1 (1~3) | day2 (0) | day2 (1~3) | day3 (0) | day3 (1~3) | day4 (0) | day4 (1~3) | day5 (0) | day5 (1~3) | day6 (0) | day6 (1~3) | day7 (0) | day7 (1~3) |
|---|---|---|---|---|---|---|---|---|---|---|---|---|---|---|---|
| Group 1 | LC-Plasma | 47 | 3 | 43 | 7 | 43 | 7 | 40 | 9 | 41 | 8 | 40 | 9 | 43 | 6 |
| | Placebo | 43 | 2 | 42 | 4 | 40 | 6 | 40 | 6 | 38 | 8 | 38 | 8 | 39 | 7 |

| No smell score | | day8 (0) | day8 (1~3) | day9 (0) | day9 (1~3) | day10 (0) | day10 (1~3) | day11 (0) | day11 (1~3) | day12 (0) | day12 (1~3) | day13 (0) | day13 (1~3) | day14 (0) | day14 (1~3) |
|---|---|---|---|---|---|---|---|---|---|---|---|---|---|---|---|
| Group 2 | LC-Plasma | 41 | 6 | 44 | 3 | 45 | 2 | 45 | 2 | 45 | 2 | 44 | 1 | 43 | 2 |
| | Placebo | 39 | 7 | 38 | 7 | 37 | 8 | 40 | 6 | 38 | 7 | 40 | 6 | 39 | 6 |

day10 p<0.05    day12 p=0.07    day13 p=0.053

**(3) No taste + no smell score**

| No taste + no smell score | | day1 (0) | day1 (1~3) | day2 (0) | day2 (1~3) | day3 (0) | day3 (1~3) | day4 (0) | day4 (1~3) | day5 (0) | day5 (1~3) | day6 (0) | day6 (1~3) | day7 (0) | day7 (1~3) |
|---|---|---|---|---|---|---|---|---|---|---|---|---|---|---|---|
| Group 1 | LC-Plasma | 44 | 6 | 42 | 8 | 42 | 8 | 39 | 10 | 40 | 9 | 40 | 8 | 43 | 6 |
| | Placebo | 43 | 2 | 42 | 4 | 40 | 6 | 40 | 6 | 38 | 8 | 38 | 8 | 39 | 7 |

| No taste + no smell score | | day8 (0) | day8 (1~3) | day9 (0) | day9 (1~3) | day10 (0) | day10 (1~3) | day11 (0) | day11 (1~3) | day12 (0) | day12 (1~3) | day13 (0) | day13 (1~3) | day14 (0) | day14 (1~3) |
|---|---|---|---|---|---|---|---|---|---|---|---|---|---|---|---|
| Group 2 | LC-Plasma | 41 | 6 | 44 | 3 | 44 | 3 | 44 | 3 | 44 | 3 | 44 | 1 | 43 | 2 |
| | Placebo | 39 | 7 | 37 | 8 | 37 | 8 | 39 | 7 | 37 | 8 | 38 | 8 | 38 | 7 |

day9 p=0.09    day10 p=0.09    day12 p=0.09    day13 p<0.05    day14 p=0.08

No taste score, no smell score, and the sum of no taste score and no smell score were confirmed to tend to be improved in the *L. lactis* strain Plasma intake group during the late infection period (on and after day 9 of intake). Thus, it was confirmed that the composition containing the *L. lactis* strain Plasma exhibits the effect as a composition for preventing or treating a chemosensory disorder such as a taste disorder or an olfactory disorder. There were no significant differences in clinical symptom scores corresponding to endpoints concerning other subjective symptoms.

[Discussion]

As a result of analyzing the proportion of patients who scored 0 (responded as asymptomatic) in each of the *L. lactis* strain Plasma intake group and the placebo intake group for taste and smell abnormalities among subjective symptoms, it was confirmed that the proportion of patients who no longer had symptoms in the *L. lactis* strain Plasma intake group increased on and after day 9 (Fig. 3). Specifically, this study demonstrated that the intake of the *L. lactis* strain Plasma accelerates the improvement of olfactory and taste abnormalities in SARS-CoV-2-positive patients.

It was confirmed that whereas the placebo intake group exhibited a significant decrease in blood pDCs due to SARS-CoV-2 infection, the *L. lactis* strain Plasma intake group maintained blood pDCs (Fig. 4).

The *L. lactis* strain Plasma intake group exhibited a greater reduction in SARS-CoV-2 viral load than that in the placebo intake group on day 4 of intake, confirming that *L. lactis* Plasma strain increases the rate of viral reduction (Fig. 5).

Although not bound by the following theory, it is believed that taking the *L. lactis* strain Plasma maintained pDCs *in vivo,* resulting in an early reduction in the SARS-CoV-2 virus and leading to early recovery from olfactory and taste disorders.

14. References

1) Daisuke Fujiwara. Lactic Acid Bacteria Activating Plasmacytoid Dendritic Cells. Experimental Medicine. 2017; 35: 191-196

2) Park A, Iwasaki A. Type I and Type III Interferons - Induction, Signaling, Evasion, and Application to Combat COVID-19. Cell Host Microbe. 2020; 27: 870-878

3) Sugimura T, Takahashi H, Jounai K, et al. Effects of oral intake of plasmacytoid dendritic cells-stimulative lactic acid bacterial strain on pathogenesis of influenza-like illness and immunological response to influenza virus. Br J Nutr. 2015; 114: 727-733

4) Rydyznski Moderbacher C, Ramirez SI, Dan JM, et al. Antigen-Specific Adaptive Immunity to SARS-CoV-2 in Acute COVID-19 and Associations with Age and Disease Severity. Cell. 2020; 183: 996-1012.e19

5) Suzuki H, Jounai K, Ohshio K, et al. Administration of plasmacytoid dendritic cell-stimulative lactic acid bacteria enhances antigen-specific immune responses. Biochem Biophys Res Commun. 2018; 503:1315-1321

6) Kato Y, Kanayama M, Yanai S, et al. Safety Evaluation of Excessive Intake of Lactococcus lactis Subsp. lactis JCM 5805: A Randomized, Double-Blind, Placebo-Controlled, Parallel-Group Trial. Food Nutr Sci. 2018; 9: 403

7) Janowitz T, Gablenz E, Pattinson D, et al. Famotidine use and quantitative symptom tracking for COVID-19 in non-hospitalised patients: A case series. Gut. 2020; 69: 1592-1597

8) He J, Guo Y, Mao R, et al. Proportion of asymptomatic coronavirus disease 2019: A systematic review and meta-analysis. J Med Virol. 2021; 93:820-830

9) Van der Sluis RM, Holm C, et al. Plasmacytoid dendritic cells during COVID-19: Ally or adversary? Cell Reports.2022; 40:1-22

**Claims**

1.   A composition for preventing or treating a chemosensory disorder, comprising a lactic acid bacterium.

2.   The composition according to claim 1, wherein the chemosensory disorder is a chemosensory disorder occurring after SARS-CoV-2 viral infection.

3.   The composition according to claim 1 or 2, wherein the chemosensory disorder is a taste disorder and/or an olfactory disorder.

4.   The composition according to claim 1 or 2, wherein the chemosensory disorder is a chemosensory disorder occurring within 7 days after viral infection.

5.   The composition according to claim 1 or 2, wherein the lactic acid bacterium is *Lactococcus lactis* subsp. *lactis* JCM5805 strain.

6. The composition according to claim 1 or 2, wherein the composition is administered orally.

7. The composition according to claim 6, wherein a dosage of the lactic acid bacterium is 20 mg or more per day.

8. The composition according to claim 6, wherein a dosage of the lactic acid bacterium is $1 \times 10^9$ or more cells per day.

9. The composition according to claim 1 or 2, which is a pharmaceutical composition or a food composition.

10. A method for preventing or treating a chemosensory disorder, comprising:
administering a prophylactically or therapeutically effective amount of a lactic acid bacterium or a composition comprising the prophylactically or therapeutically effective amount of the lactic acid bacterium to a subject in need thereof.

11. Use of a lactic acid bacterium for the manufacture of a composition for preventing or treating a chemosensory disorder.

**Fig.1**

Start date of the intake of study food
(observation start date)

Group A: *L. lactis* strain Plasma 200 mg/day intake group

Screening ---→ Obtaining consent ---→ Enrollment, Allocation

Group B: placebo intake group

±1 day ±1 day ±1 day

Recording subjective symptoms, vital sign, and study food intake condition (diary of study subject)

Conducting safety monitoring

Observation point 1,
(day1/baseline),
History taking,
Physical findings,
Blood test,
PCR test

Observation
point 2,
(day4),
History taking,
Physical findings,
Blood test,
PCR test

Observation
point 3,
(day8),
History taking,
Physical findings,
Blood test,
PCR test

Observation
point 4
(day14)

# Fig.2

Evaluation of eligibility (626)

Exclusion (514)
- Not satisfied eligibility (439)
- Wanted to be treated at home (21)
- Institutionalized at a facility other than care facilities where the study had been conducted. (1)
- No consent (53)

Temporary enrollment (112)

Exclusion (12)
- Found out not to satisfy the eligibility. (2)
- Declined to participate in the study. (1)
- Institutionalized at a facility other than the care facility where the study had been conducted. (2)
- Refused to be institutionalized to the care facility where the study had been conducted. (3)
- Wanted to delay institutionalization date. (1)
- Because the leaving date from the care facility is delayed in view of the onset date and the enrollment date. (1)
- Duplicate enrollment of the same subject (2)

Definitive enrollment/randomized allocation (100)

L. lactis strain Plasma intake group (51)

Exclusion (0)

Safety analysis set (51), Discontinuation (1), Dropout (1)

Exclusion (1)
- Found out to not satisfy inclusion criterion 1 (1)

FAS (50), Discontinuation (1), Dropout (1)

Exclusion (5)
- Study food intake rate of less than 75% (4)
- Violation of prohibited concomitant therapy (use of intestinal regulator) (1)

PPS (45) Discontinuation (0), Dropout (0)

Placebo intake group (49)

Exclusion (1)
- Dropout before the start of the study due to withdrawal from study participation (1)

Safety analysis set (48) Discontinuation (0), Dropout (0)

Exclusion (2)
- Found out not to satisfy inclusion criterion 1 (2)

FAS (46) Discontinuation (0), Dropout (0)

Exclusion (0)

PPS (46) Discontinuation (0), Dropout (0)

Fig.3

# Fig.4

∗:P<0.05 (between groups)

Percentage change in proportion of pDCs (%)

— L. lactis strain Plasma intake group
--●-- Placebo intake group

P=0.32

Time after the start of medication (days)

# Fig.5

*:P<0.05 (vs within a group day 1)

L. lactis strain Plasma intake group
Placebo intake group

Percentage change in SARS-CoV-2 viral load (%)

Time after the start of medication (days)

## INTERNATIONAL SEARCH REPORT

| International application No. |
|---|
| **PCT/JP2024/013573** |

### A. CLASSIFICATION OF SUBJECT MATTER

*A61K 35/744*(2015.01)i; *A61P 27/00*(2006.01)i; *A61P 31/14*(2006.01)i
FI: A61K35/744; A61P31/14; A61P27/00

According to International Patent Classification (IPC) or to both national classification and IPC

### B. FIELDS SEARCHED

Minimum documentation searched (classification system followed by classification symbols)

A61K35/744; A61P27/00; A61P31/14

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | ENDAM, Leandra Mfuna et al. Intranasal application of Lactococcus lactis W 136 bacteria early in SARS-Cov-2 infection may have a beneficial immunomodulatory effect: a proof-of-concept study. medRxiv. 2021, pp. 1-26, https://doi.org/10.1101/2021.04.18.21255699 abstract, table 2 | 1-4, 6-11 |
| Y | | 5 |
| Y | YAMAMOTO, Kazuko et al. Efficacy of Lactococcus lactis strain plasma (LC-Plasma) in easing symptoms in patients with mild COVID-19: protocol for an exploratory, multicentre, double-blinded, randomized controlled trial (PLATEAU study). BMJ Open. 2022, vol. 12 , e061172 (pp. 1-7) abstract, etc. | 5 |
| A | JP 2022-8060 A (NEOPHARMA JAPAN CO., LTD.) 13 January 2022 (2022-01-13) examples, etc. | 1-11 |

☑ Further documents are listed in the continuation of Box C.  ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **11 June 2024** | **25 June 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT

| International application No. |
| --- |
| **PCT/JP2024/013573** |

### C. DOCUMENTS CONSIDERED TO BE RELEVANT

| Category* | Citation of document, with indication, where appropriate, of the relevant passages | Relevant to claim No. |
| --- | --- | --- |
| A | MELE, Dalila et al. High Frequencies of Functional Virus-Specific CD4+ T Cells in SARS-CoV-2 Subjects With Olfactory and Taste Disorders. Frontiers in Immunology. 2021, vol. 12, article.748881 (pp. 1-11), ISSN1664-3224<br>　　abstract, etc. | 1-11 |
| A | 藤原大介, スポンサードシンポジウム8　宿主免疫調節の新たなアプローチ　SS8-2 自然免疫を活性化する乳酸菌の感染症に対する効果, 日本化学療法学会雑誌 第70回日本化学療法学会総会特集号, vol. 70 suppl-A, 2022, p. 249, (FUJIWARA, Daisuke), non-official translation (Sponsored Symposium 8: New approaches to host immune regulation SS8-2. The effect of lactic acid bacteria that activate Innate immunity against infectious diseases. Japanese Journal of Chemotherapy: Special Issue of the 70th Annual Meeting of the Japanese Society of Chemotherapy.)<br>　　2nd to 3rd paragraphs | 1-11 |

Form PCT/ISA/210 (second sheet) (July 2022)

INTERNATIONAL SEARCH REPORT
Information on patent family members

International application No.

**PCT/JP2024/013573**

| Patent document cited in search report | Publication date (day/month/year) | Patent family member(s) | Publication date (day/month/year) |
|---|---|---|---|
| JP 2022-8060 A | 13 January 2022 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63493874

### Non-patent literature cited in the description

- **DAISUKE FUJIWARA**. Lactic Acid Bacteria Activating Plasmacytoid Dendritic Cells. *Experimental Medicine*, 2017, vol. 35, 191-196
- **PARK A** ; **IWASAKI A.** Type I and Type III Interferons - Induction, Signaling, Evasion, and Application to Combat COVID-19. *Cell Host Microbe.*, 2020, vol. 27, 870-878
- **SUGIMURA T** ; **TAKAHASHI H** ; **JOUNAI K et al.** Effects of oral intake of plasmacytoid dendritic cells-stimulative lactic acid bacterial strain on pathogenesis of influenza-like illness and immunological response to influenza virus. *Br J Nutr.*, 2015, vol. 114, 727-733
- **RYDYZNSKI MODERBACHER C** ; **RAMIREZ SI** ; **DAN JM et al.** Antigen-Specific Adaptive Immunity to SARS-CoV-2 in Acute COVID-19 and Associations with Age and Disease Severity. *Cell*, 2020, vol. 183, 996-1012.e19
- **SUZUKI H** ; **JOUNAI K** ; **OHSHIO K et al.** Administration of plasmacytoid dendritic cell-stimulative lactic acid bacteria enhances antigen-specific immune responses. *Biochem Biophys Res Commun.*, 2018, vol. 503, 1315-1321
- **KATO Y** ; **KANAYAMA M** ; **YANAI S et al.** Safety Evaluation of Excessive Intake of Lactococcus lactis Subsp. lactis JCM 5805: A Randomized, Double-Blind, Placebo-Controlled, Parallel-Group Trial. *Food Nutr Sci.*, 2018, vol. 9, 403
- **JANOWITZ T** ; **GABLENZ E** ; **PATTINSON D et al.** Famotidine use and quantitative symptom tracking for COVID-19 in non-hospitalised patients: A case series. *Gut*, 2020, vol. 69, 1592-1597
- **HE J** ; **GUO Y** ; **MAO R et al.** Proportion of asymptomatic coronavirus disease 2019: A systematic review and meta-analysis. *J Med Virol.*, 2021, vol. 93, 820-830
- **VAN DER SLUIS RM** ; **HOLM C et al.** Plasmacytoid dendritic cells during COVID-19: Ally or adversary?. *Cell Reports*, 2022, vol. 40, 1-22